# EUROPÄISCHE PATENTSCHRIFT

(11) **EP 2 132 174 B1**
(45) Veröffentlichungstag und Bekanntmachung des Hinweises auf die Patenterteilung: **01.02.2012**
(21) Anmeldenummer: 08716267.3
(22) Anmeldetag: 05.03.2008
(51) Int. Cl.: C07D 211/26, C07D 211/34, C07D 211/60, C07D 295/18, C07D 295/20, C07C 257/18, C07C 311/37, A61K 31/445, A61P 35/00, A61P 35/04

(54) **META-SUBSTITUIERTE PHENYLSULFONYLAMIDE SEKUNDÄRER AMINOSÄUREAMIDE ALS HEMMSTOFFE DER MATRIPTASE ZUR HEMMUNG DES TUMORWACHSTUMS UND/ODER DER METASTASIERUNG VON TUMOREN**
META-SUBSTITUTED PHENYL SULFONYL AMIDES OF SECONDARY AMINO ACID AMIDES, THE PRODUCTION THEREOF, AND USE THEREOF AS MATRIPTASE INHIBITORS
AMIDES D'AMINOACIDES SECONDAIRES DE PHÉNYLSULFONYLAMIDE MÉTA-SUBSTITUÉ, LEUR PRODUCTION ET LEUR UTILISATION COMME INHIBITEURS DE LA MATRIPTASE

(30) Priorität: 06.03.2007 DE 102007010815
(43) Veröffentlichungstag der Anmeldung: 16.12.2009
(73) Patentinhaber: The Medicines Company (Leipzig) GmbH, 04103 Leipzig (DE)
(72) Erfinder: STEINMETZER, Torsten, 07743 Jena (DE); SCHWEINITZ, Andrea, 07749 Jena (DE); DÖNNECKE, Daniel, Victoria BC, V8Z 5L8 (CA)
(74) Vertreter: Bösl, Raphael Konrad
(86) Internationale Anmeldenummer: PCT/EP2008/001750
(87) Internationale Veröffentlichungsnummer: WO 2008/107176

(56) Entgegenhaltungen:
- WO-A-2004/101507
- STEINMETZER, TORSTEN ET AL: "Secondary amides of sulfonylated 3-amidinophenylalanine. New potent and selective inhibitors of matriptase" JOURNAL OF MEDICINAL CHEMISTRY, Bd. 49, Nr. 14, 2006, Seiten 4116-4126, XP002487483 in der Anmeldung erwähnt

## Beschreibung

Die vorliegende Erfindung betrifft meta-substituierte Phenylsulfonylamide sekundärer Aminosäureamide gemäß der allgemeinen Formel (I), (II) oder (III), ihre Herstellung und Verwendung als Hemmstoffe der Matriptase, insbesondere deren Verwendung als Arzneimittel zur Hemmung des Tumorwachstums und/oder der Metastasierung von Tumoren.

Proteasen regulieren zahlreiche physiologische Prozesse, die das Wachstum und die Metastasierung von Tumorzellen ermöglichen bzw. stimulieren. Das betrifft insbesondere den proteolytischen Abbau der die Tumorzellen umgebenden extrazellulären Matrixproteine, durch den die Invasion der von Tumoren abgesiedelten Tumorzellen in angrenzende Gewebe und in das Lymph- bzw. Blutsystem ermöglicht wird. Proteasen sind auch an der Aktivierung von Wachstumsfaktoren beteiligt, die z.B. die Proliferation von Tumorzellen oder die Angiogenese stimulieren und damit das Tumorwachstum ermöglichen. Zu diesen proteolytischen Enzymen gehören verschiedene Matrix-Metalloproteasen, membrangebundene Metalloproteasen, lysosomale Cysteinproteasen und eine Vielzahl von Serinproteasen, wie z.B. Urokinase, Plasmin, Elastase, Thrombin oder Cathepsin G und auch die Type-II-Transmembran-Serinprotease Matriptase.

Es gibt zahlreiche Versuche, durch den Einsatz von Proteaseinhibitoren das Wachstum und die Metastasierung von Tumoren zu hemmen, doch zeigten Versuche mit Hemmstoffen der Matrix-Metalloproteasen bisher kaum Wirkung in klinischen Studien (Coussens et al., Science 295, 2387-2392, 2002).

Matriptase ist eine trypsinartige Serinprotease, die ursprünglich aus Brustkrebszellen isoliert wurde und bevorzugt C-terminal Peptidbindungen der basischen Aminosäure Arginin spaltet.

Für Matriptase werden in der Literatur auch die Bezeichnungen MT-SP1 (membran-type serine protease 1), TADG-15 (tumor-associated differentially expressed gene-15) oder ST14 (suppressor of tumorigenicity 14)/SNC19 verwendet. Der Begriff Matriptase umfasst auch die durch Spaltung der normalerweise membranständigen Protease entstandenen verkürzten Proteinformen mit proteolytischer Aktivität, die gegebenenfalls nicht mehr mem.branständig sind.

1998 wurde das Gen der Matriptase als putativer Tumorsuppressor durch ein subtraktives Hybridisierungsverfahren, bei dem gesundes und kanzerogenes Darmgewebe verwendet wurden, kloniert (Zhang et al. Cytogenet. Cell Genet. 83, 56-57, 1998).

Matriptase und MT-SP1 besitzen die gleiche cDNA. Allerdings ist aufgrund alternativen Splicings die Proteinsequenz der Matriptase im Vergleich zur MT-SP1 um 172 Aminosäuren am N-Terminus verkürzt. Das Gen für MT-SP1 wurde aus einer epithelialen Zelllinie eines Prostatatumors isoliert.

Im Rahmen der vorliegenden Erfindung bezeichnet der Begriff "Matriptase" jedes trypsinartige Protein, das von den Gensequenzen mit den Eintragsnummer AF118224, AF133086, BANKIt257050 und NM021978 (GenBank/EBI Data Bank) abgeleitet ist und bereits früher beschrieben wurde (Takeuchi et al., Proc. Natl. Acad. Sci. USA 96, 11054-11061, 1999; Lin et al., J. Biol. Chem. 274, 18231-18236, 1999).

Das Enzym ist mittels einer Transmembran-Domäne in der Membran von Epithel- oder Krebszellen verankert, wobei die Serinproteasedomäne der Matriptase auf der Zelloberfläche und damit im extrazellulären Raum lokalisiert ist. Deshalb geht man davon aus, dass Matriptase an der Proliferation und Metastasierung von Brustkrebszellen durch Ab- bzw. Umbau extrazellulärer Matrixproteine, der Aktivierung von latenten Wachstumsfaktoren und anderen proteolytischen Kaskaden beteiligt ist (Shi et al., Cancer Res. 53, 1409-1415, 1993; Lin et al., J. Biol. Chem. 272, 9147-9152, 1997).

Matriptase konnte auch aus menschlicher Milch isoliert werden, doch lag sie in diesem Fall fast vollständig als proteolytisch inaktiver Komplex mit dem endogenen Inhibitor HAI-1 vor (Lin et al., J. Biol. Chem. 274, 18237-18242, 1999). Im Gegensatz dazu liegt die Matriptase aus Brustkrebszellen weitestgehend in unkomplexierter und dadurch katalytisch wirksamer Form vor und nur ein geringer Teil ist an HAI-1 gebunden.

Inzwischen wurden potentielle Substrate der Matriptase beschrieben, unter anderem spaltet sie das Polyprotein Profilaggrin in Fillaggrin-Monomere, die notwendig für eine normale Epidermisbildung sind. Zusätzlich kann Matriptase die Proform des Hepatocyten-Wachstumsfaktor (HGF) aktivieren, der auch als Scattering-Faktor bezeichnet wird. Pro-HGF wird von Krebs- oder Stromazellen in inaktiver Form als einkettiges Protein sekretiert und wird im extrazellulären Raum durch Spaltung C-terminal des Arg495 in die aktive zweikettige Form (HGF) umgewandelt. Durch die Bindung von HGF wird der Zelloberflächenrezeptor c-Met aktiviert und an bestimmten Tyrosinresten phosphoryliert. Kürzlich wurde eine enge Korrelation zwischen einer hohen Expression von c-Met, Matriptase und HAI-1 und einer schlechten Prognose bei Brustkrebspatienten nachgewiesen (Kang et al., Cancer Res. 63, 1101-1105, 2003). Auch bei der Untersuchung von Ovarialtumoren konnte gezeigt werden, dass Matriptase in verstärktem Maße exprimiert wird. Dabei wurde gefunden, dass im Gegensatz zu Tumoren des Typs I/II vor allem in fortgeschrittenen Tumoren des Typs III/IV Matriptase fast ausschließlich ohne HAI-1 exprimiert wird. Dies deutet darauf hin, dass im fortgeschrittenen Stadium ein Ungleichgewicht zwischen Matriptase und dem Inhibitor HAI-1 besteht, wodurch die proteolytische Aktivität der Matriptase und dadurch wahrscheinlich auch das invasive Potential der Tumorzellen verstärkt wird (Oberst et al., Clin. Cancer Res. 8, 1101-1107, 2002).

Neben der Aktivierung von Pro-HGF ist Matriptase möglicherweise auch an der Aktivierung der Plasminogenaktivator-Kaskade beteiligt. So ist Matriptase in der Lage, Pro-Urokinase zu Urokinase (uPA) zu aktivieren (Lee et al., J. Biol. Chem. 275, 36720-36725, 2000; Takeuchi et al., J. Biol. Chem. 275, 26333-26342, 2000), die Plasminogen in Plasmin umwandelt. Plasmin ist der prinzipielle Aktivator der Matrix-Metalloproteasen, die am Abbau extrazellulärer Matrixproteine beteiligt sind, was als Voraussetzung der Metastasierung angesehen wird.

Von Ihara et al. (J. Biol. Chem. 277, 16960-16967, 2002) konnte gezeigt werden, dass Magenkrebszellen verstärkt β1-6-N-Acetylglucosaminyltransferase (GnT-V) exprimieren, die in der Lage ist, Matriptase zu glycosylieren. Durch diese Modifizierung wird die Matriptase abbaustabiler und liegt in erhöhter Konzentration in proteolytisch aktiver Form vor.

Aus diesen Befunden kann abgeleitet werden, dass mit der Entwicklung eines wirksamen und selektiven Hemmstoffs der Matriptase eine Möglichkeit besteht, die Proliferation von Tumoren und ihre Metastasierung zu hemmen. Obwohl inzwischen auch die Röntgenstruktur der katalytischen Domäne der Matriptase im Komplex mit Benzamidin und dem Rinderpankreas-Trypsininhibitor aufgeklärt werden konnte, sind bisher allerdings erst wenige Hemmstoffe der Matriptase bekannt (Friedrich et al., J. Biol. Chem. 277, 2160-2168, 2002).

Von Enyedy et al. (J. Med. Chem. 44, 1349-1355, 2001) wurden Bis-Benzamidine beschrieben, wobei der wirksamste Inhibitor einen Kᵢ-Wert von 0,19 µM aufweist.

Die internationale Patentanmeldung WO 01/97794 beschreibt ein Verfahren zur Hemmung einer Carcinomprogression, bei der Matriptase eine Rolle spielt. Dabei werden Verbindungen eingesetzt, die zwei Gruppen enthalten, die in der Lage sind, bei einem physiologischen pH-Wert positiv geladen zu sein. Dabei sind diese Gruppen durch eine chemische Struktureinheit miteinander verbunden, die eine Länge von 5 bis 30, bevorzugt 15 bis 24 Angstrom aufweist. Als positiv geladene Gruppen werden Amino-, Amidino-, Guanidinogruppe sowie eine aus der Amidino- bzw. Guanidinogruppe abgeleitete cyclische Gruppe offenbart. Aminosäurederivate sind in der WO 01/97794 nicht erwähnt, insbesondere demgemäß keine sulfonylierten Aminosäurederivate. Vielmehr unterscheiden sich die in der WO 01/97794 explizit offenbarten Verbindungen grundsätzlich von den im Rahmen der vorliegenden Erfindung beanspruchten Verbindungen.

In WO 02/20475 sind Tripeptidaldehyde mit C-terminalem Arginal veröffentlicht. Nach Vorinkubation von Matriptase mit diesen Inhibitoren über einen Zeitraum von 30 Minuten wurden IC₅₀-Werte kleiner 100 nM für die wirksamsten Verbindungen bestimmt, exakte Hemmkonstanten wurden allerdings nicht angegeben. Diese Inhibitoren binden vermutlich kovalent unter Ausbildung eines Halbacetals an Matriptase. Im Falle der Entwicklung von Hemmstoffen für andere trypsinartige Serinproteasen, wie z.B. Thrombin oder Faktor Xa, wurde jedoch gezeigt, dass solche übergangszustandsanalogen Peptidaldehyde für die Entwicklung eines medizinisch einsetzbaren Wirkstoffs nicht geeignet sind. Kürzlich wurde gezeigt, dass durch intraperitoneale Gabe des Arginalderivates CVS-3983 das Wachstum eines Androgen-unabhängigen Tumors reduziert werden kann (Galkin et al., The Prostate 61, 228-235, 2004). Von diesen Autoren wurden weitere substratanaloge Tripeptid-Derivate beschrieben, bei denen das C-terminale Arginal durch eine Reihe von P1-Resten ausgetauscht wurde, die eine Bindung in die S1-Tasche des Enzyms ermöglichen (WO 2004/058688).

Von Long et al. (Bioorganic. Med. Chem. Lett. 11, 2515-2519, 2001) wurde die Synthese eines bizyklischen Peptides aus 14 Aminosäuren veröffentlicht, das ursprünglich aus dem Samen von Sonnenblumen isoliert wurde. Das Peptid inhibiert Matriptase mit einer Hemmkonstante von 0,92 nM, doch ist davon auszugehen, dass diese Struktur aufgrund ihrer geringen Spezifität für eine klinische Entwicklung nicht geeignet ist. Weitere redoxstabilisierte Derivate dieses Peptides mit verminderter Aktivität wurden kürzlich in Jiang et al., Organic Lett. 9, 9-12, 2007 beschrieben.

In der WO 2004/101507 werden sulfonylierte Derivate des 3-Amidinophenylalanins beschrieben, die C-terminal mit einem sekundären Amid modifiziert sind, das notwendigerweise einen weiteren basischen Substituenten besitzt. Die wirksamsten Verbindungen hemmen Matriptase mit Kᵢ-Werten < 5 nM (Steinmetzer et al., J. Med. Chem. 49, 4116-4126, 2006). Ein Nachteil dieser besonders wirksamen Verbindungen mit Ki-Werten < 10 nM besteht darin, dass sie aufgrund ihrer drei geladenen stark basischen Gruppen im N- und C-terminalen Rest, sowie im zentralen Aminosäurebaustein und der damit verbundenen Hydrophilie nach oraler Applikation an Versuchstieren nur in sehr geringen Mengen aufgenommen wurden und nach intravenöser Applikation an Ratten in den meisten Fällen nur eine sehr kurze Verweildauer in der Zirkulation besitzen.

Eine der vorliegenden Erfindung zugrunde liegende Aufgabe war es daher, einen auch für therapeutische Anwendungen geeigneten Wirkstoff mit verbesserten Eigenschaften, insbesondere für die orale Gabe bereitzustellen, der Matriptase mit hoher Aktivität und Spezifität hemmt und eine reduzierte Basizität besitzt.

Überraschenderweise wurde nun gefunden, dass meta-substituierte Phenylsulfonylamide sekundärer Aminosäureamide als Hemmstoffe der Matriptase wirken, auch wenn sie keinen basischen Rest mehr im N-terminalen Sulfonylrest oder im C-terminalen sekundären Amidrest besitzen.

Zusätzlich wurde überraschenderweise gefunden, dass man in diesem Strukturtyp das zentrale 3-Amidinophenylalanin durch das weniger stark basische 3-Aminomethylphenylalanin ersetzen kann und dennoch wirksame Matriptasehemmstoffe erhält.

Ein Gegenstand der vorliegenden Erfindung ist daher eine Verbindung gemäß Formel (I) 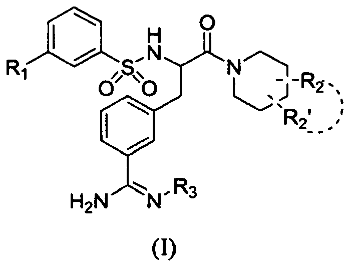 oder ein Salz dieser Verbindung, wobei
R₁ eine gegebenenfalls ein- oder mehrfach substituierte Ringstruktur ist ausgewählt aus einem Arylrest oder Heteroarylrest, die auch teilweise oder vollständig hydriert sein können; und
R₂ und R₂', sofern einzeln oder zusammen vorhanden, unabhängig voneinander sind ein verzweigter oder geradkettiger Alkylrest mit 1-6 Kohlenstoffatomen, wobei ein oder mehrere Methylengruppen durch Heteroatome ausgetauscht sein können; ein verzweigter oder geradkettiger Aminoalkylrest oder Guanidinoalkylrest mit 1-6 Kohlenstoffatomen; oder -(CH₂)ₘ-C(=O)-NHR₄ mit m gleich eine ganze Zahl von 0 bis 4 und R₄ ein Wasserstoff oder ein -(CH₂)ₖ-CH₃ Rest mit k gleich eine ganze Zahl von 0 bis 3; oder R₂ und R₂' bilden eine Ringstruktur, die zusammen mit dem Piperidid folgendes Strukturelement bilden: 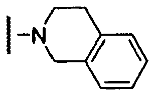 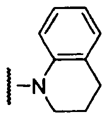 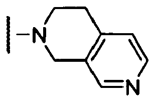 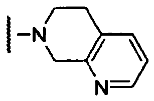 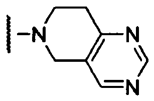 oder
R₁ ist ein verzweigter oder geradkettiger Aminoalkylrest, Hydroxyalkylrest mit 1 bis 6 Kohlenstoffatomen; oder ein 3-Azetidin-C(=O)-NH-Rest; und
R₂ und R₂', sofern einzeln oder zusammen vorhanden, unabhängig voneinander sind ein verzweigter oder geradkettiger Aminoalkylrest oder Guanidinoalkylrest mit 1-6 Kohlenstoffatomen; oder R₂ ist -(CH₂)ₘC(=O)-NHR₄ mit m gleich eine ganze Zahl von 0 bis 4 und R₄ ein Wasserstoff oder ein -(CH₂)ₖ-CH₃ Rest mit k gleich eine ganze Zahl von 0 bis 3;
oder
R₁ ist ein H₂N-(CH₂)ₙ-C(=O)-NH-Rest, ein HO-(CH₂)ₙ-C(=O)-NH-Rest mit n gleich eine ganze Zahl von 1 bis 4; oder ein 3-Azetidin-C(=O)-NH-Rest; und
R₂ und R₂', sofern einzeln oder zusammen vorhanden, unabhängig voneinander sind ein nicht basischer Rest; oder R₂ und R₂' bilden eine Ringstruktur, die zusammen mit dem Piperidid eines der oben genannten Strukturelemente bilden;
und
R₃ ist in allen obigen Fällen ein Wasserstoff, eine Hydroxylgruppe, eine Alkoxygruppe, eine Acetyloxygruppe oder eine Alkyloxycarbonylgruppe, wobei der Alkylrest 1-6 Kohlenstoffatome besitzt.

Bevorzugte Verbindung sind solche Verbindungen, bei denen R₂' nicht vorhanden ist und bei denen
R₁ ausgewählt ist aus einem gegebenenfalls ein- oder mehrfach substituierten Phenylrest, Pyridylrest, Pyrimidinrest, Indolrest, Tetrahydropyridylrest, Piperidinonrest oder Pyridazinonrest; und
R₂ ist ein geradkettiger Aminoalkylrest mit 1-6 Kohlenstoffatomen; oder -(CH₂)ₘ-C(=O)-NHR₄ mit m gleich eine ganze Zahl von 0 bis 4 und R₄ ein Wasserstoff oder ein -(CH₂)ₖ-CH₃ Rest mit k gleich eine ganze Zahl von 0 bis 3;
oder
R₁ und R₂ sind ein geradkettiger Aminoalkylrest mit 1 bis 6 Kohlenstoffatomen;
oder
R₁ ist ein H₂N-(CH₂)ₙ-C(=O)-NH-Rest mit n gleich eine ganze Zahl von 1 bis 4; und
R₂ ist ausgewählt aus folgenden Resten:
-(CH₂)ₘ C(=O)-NHR₄ mit m gleich eine ganze Zahl von 0 bis 4, und R₄ ein Wasserstoff oder ein -(CH₂)ₖ-CH₃ Rest mit k gleich eine ganze Zahl von 0 bis 3; oder
-(CH₂)ₒ-C(=O)-OR₅ mit o eine ganze Zahl von 1 bis 6 und R₅ ein Wasserstoff oder eine geradkettige Alkylgruppe mit 1 bis 4 Kohlenstoffatomen;
und
R₃ ist in allen obigen Fällen ein Wasserstoff, eine Hydroxylgruppe, eine Alkoxygruppe, eine Acetyloxygruppe oder eine Alkyloxycarbonylgruppe, wobei der Alkylrest 1-6 Kohlenstoffatome besitzt.

Besonders bevorzugt ist eine Verbindung gemäß Formel (I), bei der der Rest R₂ in meta-oder para-Position vorliegt. Ebenfalls behandelt werden Verbindungen gemäß Formel (I), bei denen, wenn sowohl R₂ wie auch R₂' vorliegen, der Rest R₂ in para-Position und der Rest R₂' in ortho- oder meta-Position vorliegen.

Des Weiteren wird eine Verbindung gemäß Formel (I) behandelt, bei der die Ringstruktur vom Rest R₁ in meta- und/oder para-Position substituiert ist.

Bevorzugte Verbindungen sind solche, bei denen die Substitution an der Ringstruktur vom Rest R₁ ausgewählt ist aus folgenden Resten: R₆-O- mit R₆ ein verzweigter oder geradkettiger Alkylrest mit 1 bis 6 Kohlenstoffatomen; (CH₃)-(CH₂)ₚ- mit p gleich eine ganze Zahl von 0 bis 6; ein Halogen; und/oder eine Aminogruppe.

Insbesondere ist die Substitution am Phenylrest aus folgenden Resten ausgewählt: R₆-O-mit R₆ ein verzweigter oder geradkettiger Alkylrest mit 1 bis 6 Kohlenstoffatomen; (CH₃)-(CH₂)ₚ- mit p gleich eine ganze Zahl von 0 bis 6; und/oder ein Halogen.

Die Substitution am Pyridylrest, am Pyrimidinrest oder am Tetrahydropyridylrest ist insbesondere ausgewählt aus folgenden Resten: (CH₃)-(CH₂)ₚ- mit p gleich eine ganze Zahl von 0 bis 6; und/oder eine Aminogruppe. Günstig ist es, wenn die Substitution am Pyrimidinrest eine (CH₃)-(CH₂)ₚ-Gruppe ist p gleich eine ganze Zahl von 0 bis 6, beispielsweise von 0 bis 2, wie 1, günstigerweise eine Methylgruppe.

Ein weiterer Gegenstand der vorliegenden Erfindung ist eine Verbindung gemäß Formel (II) 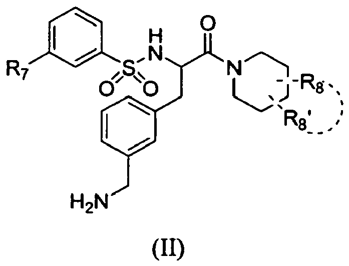 oder ein Salz dieser Verbindung, wobei
R₇ eine gegebenenfalls ein oder mehrfach substituierte Ringstruktur ist ausgewählt aus einem Arylrest oder Heteroarylrest, die gegebenenfalls teilweise oder vollständig aufhydriert ist; ein verzweigter oder unverzweigter Alkylrest mit 1-8 Kohlenstoffatomen, wobei eine oder mehrere Methylengruppen durch Heteroatome ausgetauscht sein können; ein H₂N-(CH₂)ᵣ-C(=O)-NH-Rest, ein HO-(CH₂)ᵣ-C(=O)-NH-Rest, ein NH₂-(CH₂)_{q}-Rest, oder ein HO-(CH₂)_{q}-Rest, mit r gleich eine ganze Zahl von 1 bis 4 und q unabhängig voneinander gleich eine ganze Zahl von 1 bis 5 ist; oder ein 3-Azetidin-C(=O)-NH-Rest; und
R₈ und R₈', sofern einzeln oder zusammen vorhanden, unabhängig voneinander sind ein verzweigter oder geradkettiger Alkylrest, mit 1-8 Kohlenstoffatomen, wobei eine oder mehrere Methylengruppen durch Heteroatome ausgetauscht sein können; ein Aminoalkylrest oder Guanidinoalkylrest mit jeweils 1-6 Kohlenstoffatomen; oder ein -(CH₂)ₘ-C(=O)-NHR₄ mit m gleich eine ganze Zahl von 0 bis 4 und R₄ ein Wasserstoff oder ein -(CH₂)ₖ-CH₃ Rest mit k gleich eine ganze Zahl von 0 bis 3; oder R₈ und R₈' bilden eine Ringstruktur, die zusammen mit dem Piperidid ein wie oben definiertes Strukturelement bilden.

Bevorzugt sind Verbindungen, bei denen R₈' nicht vorhanden ist und
R₇ eine gegebenenfalls ein oder mehrfach substituierte Ringstruktur ist ausgewählt aus einem Phenylrest, Pyridylrest, Pyrimidinrest, Indolrest, Tetrahydropyridylrest, Piperidinonrest oder Pyridazinonrest; ein H₂N-(CH₂)ᵣ-C(=O)-NH-Rest, mit r gleich eine ganze Zahl von 1 bis 4; und
R₈ ist ein Aminoalkylrest mit jeweils 1-6 Kohlenstoffatomen.

Beispielsweise ist R₇ ein gegebenenfalls einfach substituierter Phenylrest oder ein NH₂-(CH₂)ᵣ-C(=O)-NH-Rest und r hat die genannte Bedeutung.

Bei weiteren beispielhaften Verbindungen steht der Rest R₈ in para-Position des Piperidinringes. Der Rest R₈' kann, falls vorhanden, dann in ortho- oder meta-Position des Piperidinringes stehen.

Bei weiteren Verbindungen ist die Substitution an R₇ vorteilhafterweise ausgewählt aus folgenden Resten: R₁₀-O- mit R₁₀ ein verzweigter oder geradkettiger Alkylrest mit 1 bis 6, beispielsweise 1 bis 4, wie 1 bis 3 Kohlenstoffatomen, oder (CH₃)-(CH₂)ₛ-mit s gleich eine ganze Zahl von 0 bis 6, beispielsweise von 0 bis 2, wie 1, oder eine Aminogruppe ist. Günstig ist es, wenn die Substitution am Phenylrest ein R₁₀-O-Rest mit R₁₀ ein verzweigter oder geradkettiger Alkylrest mit 1 bis 6, unter anderem 1 bis 4, beispielsweise 1 bis 3 Kohlenstoffatomen, wie 1 Kohlenstoffatom, ist.

Ein weiterer Gegenstand der vorliegenden Erfindung ist eine Verbindung gemäß Formel (III) 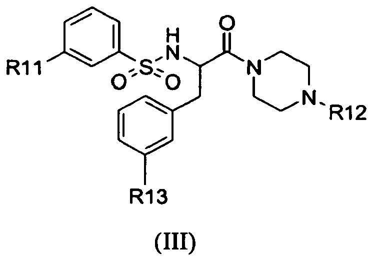 oder ein Salz dieser Verbindung, wobei
R11 ein NH₂-(CH₂)ᵥ-C(=O)-NH-Rest ist mit v gleich eine ganze Zahl von 1 bis 4; ein 3-Azetidin-C(=O)-NH-Rest, eine gegebenenfalls ein- oder mehrfach substituierte Ringstruktur ist ausgewählt aus einem Arylrest oder Heteroarylrest, die auch teilweise oder vollständig hydriert sein können;
R12 ist ein Wasserstoff; ein verzweigter oder geradkettiger Aminoalkylrest mit 1 bis 6 Kohlenstoffatomen; eine -C(=O)-O-R14 Gruppe, -C(=O)-R14 Gruppe oder -C(=O)-NH-R14 Gruppe mit jeweils R14 unabhängig voneinander gleich ein Alkylrest mit 1 bis 6 Kohlenstoffatomen; oder ein Aralkylrest oder Heteroaralkylrest mit jeweils 4-12 Kohlenstoffatomen in deren Aryl- oder Heteroarylsegment, wobei das Heteroarylsegment des Heteroaralkylrestes 1 bis 2 Heteroatome enthalten kann, und mit jeweils 1 bis 6 Kohlenstoffatomen im Alkylrest; und
R13 ist ein Aminomethylrest oder ein substituierter oder unsubstituierter Amidinorest der Formel -C(=NR15)NH₂ mit R15 gleich Wasserstoff, eine Hydroxylgruppe, eine Alkoxygruppe, eine Acetyloxygruppe oder eine Alkyloxycarbonylgruppe, wobei der Alkylrest 1-6 Kohlenstoffatome besitzt.

Bevorzugte Verbindungen sind Verbindungen, bei denen
R11 ein NH₂-(CH₂)ᵥ-C(=O)-NH-Rest ist mit v gleich eine ganze Zahl von 1 bis 4;
R12 ist ein Wasserstoff; ein geradkettiger Aminoalkylrest mit 1 bis 6 Kohlenstoffatomen; oder eine -C(=O)-O-R14 Gruppe mit jeweils R14 unabhängig voneinander gleich ein geradkettiger Alkylrest mit 1 bis 6 Kohlenstoffatomen; oder ein Benzylrest; und
R13 ist ein Aminomethylrest oder ein substituierter oder unsubstituierter Amidinorest der Formel -C(=NR15)NH₂ mit R15 gleich Wasserstoff, eine Hydroxylgruppe, eine Alkoxygruppe, eine Acetyloxygruppe oder eine Alkyloxycarbonylgruppe, wobei der Alkylrest 1-6 Kohlenstoffatome besitzt.

In einer besonderen Ausführungsform können die erfindungsgemäßen Verbindungen entweder direkt über funktionelle Gruppen oder indirekt mit Hilfe eines Linkers an Polyethylenglykol (PEG), beispielsweise an PEG mit 5-10 kDa nach dem Fachmann bekannten Verfahren kovalent gekoppelt werden (PEGylierung). Hierdurch wird beispielsweise eine längere Halbwertszeit der entsprechenden Verbindung im Patienten erreicht. Als Linker eignet sich beispielsweise eine Alkylkette mit z. B. 2-6 Kohlenstoffatomen und entsprechenden funktionellen Gruppen, wie in den Beispielen aufgezeigt ist. Beispielsweise wird eine Polyethylenglykolkette an die erfindungsgemäßen Verbindungen über die Reste R₁, R₂, R₂', R₇, R₈, R₈', R11 und/oder R12, beispielsweise mit Hilfe einer Alkylkette mit 2-6 Kohlenstoffatomen und einer Amidbindung, kovalent gekoppelt.

Bevorzugt ist daher an eine erfindungsgemäße Verbindung gegebenenfalls mit Hilfe eines Linkers eine Polyethylenglykol-Kette kovalent gekoppelt.

Im Allgemeinen sind Verbindungen mit zentraler L-Konfiguration vorteilhaft. Als geeignete Herstellungsverfahren eignen sich hierbei folgende Verfahren, die auch zur Herstellung von Razematen geeignet sind, wobei für die Synthese vorteilhafterweise enantiomerenreines 3-Cyanophenylalanin verwendet wird.

Bevorzugt liegt daher in den erfindungsgemäßen Verbindungen die als zentrales Strukturmotif in den Formeln (I), (II) und (III) vorhandene Aminosäure in der (L)-Konfiguration vor.

Besonders günstige Verbindungen, die im Rahmen der vorliegenden Erfindung beschrieben werden, sind die Verbindungen gemäß den Beispielen oder im Rahmen der allgemeinen Formel (I), (II) oder (III) von den einzelnen Beispielen abgeleitete Verbindungen. Vor allem sind hierbei die Verbindungen oder Verbindungsklassen mit einem Ki-Wert kleiner gleich 100 nM, vorteilhafterweise kleiner gleich 65 nM, beispielsweise kleiner 31 nM, wie kleiner 15 nM günstig.

Bevorzugt ist daher eine Verbindung gemäß Formel (I), wobei die Verbindung ausgewählt ist aus einer der folgenden Strukturen:
3-(3,4-Dimethoxyphenyl)phenylsulfonyl-L-Phenylalanin(3-Amidino)-4-(2-Aminoethyl)Piperidid,
3-(Phenyl)phenylsulfonyl-L-Phenylalanin(3-Amidino)-4-(2-Aminoethyl)Piperidid,
3-(4-Methoxyphenyl)phenylsulfonyl-L-Phenylalanin(3-Amidino)-4-(2-Aminoethyl)Piperidid,
3-(4-Ethoxyphenyl)phenylsulfonyl-L-Phenylalanin(3-Amidino)-4-(2-Aminoethyl)Piperidid,
3-(4-Ethylphenyl)phenylsulfonyl-L-Phenylalanin(3-Amidino)-4-(2-Aminoethyl)Piperidid,
3-(4-Isopropoxyphenyl)phenylsulfonyl-L-Phenylalanin(3-Amidino)-4-(2-Aminoethyl)Piperidid,
3-(4-Chlorphenyl)phenylsulfonyl-L-Phenylalanin(3-Amidino)-4-(2-Aminoethyl)Piperidid,
3-(3-Chlorphenyl)phenylsulfonyl-L-Phenylalanin(3-Amidino)-4-(2-Aminoethyl)Piperidid,
3-(2-Chlorphenyl)phenylsulfonyl-L-Phenylalanin(3-Amidino)-4-(2-Aminoethyl)Piperidid,
3-(4-Pyridyl)phenylsulfonyl-L-Phenylalanin(3-Amidino)-4-(2-Aminoethyl)Piperidid,
3-(3-Pyridyl)phenylsulfonyl-L-Phenylalanin(3-Amidino)-4-(2-Aminoethyl)Piperidid,
3-(2-Methyl-4-pyrimidinyl)phenylsulfonyl-L-Phenylalanin(3-Amidino)-4-(2-Aminoethyl)Piperidid,
3-(5-Indolyl)phenylsulfonyl-L-Phenylalanin(3-Amidino)-4-(2-Aminoethyl)Piperidid,
3-(4-Amino-3-pyridyl)phenylsulfonyl-L-Phenylalanin(3-Amidino)-4-(2-Aminoethyl)Piperidid,
3-(4-Amino-3-Tetrahydropyridyl)phenylsulfonyl-L-Phenylalanin(3-Amidino)-4-(2-Aminoethyl)Piperidid,
3-(1-Aminobutyl)-Phenylsulfonyl-L-Phenylalanin(3-Amidino)-4-(2-Aminoethyl)Piperidid,
3-(H-β-Alanyl-NH)phenylsulfonyl-L-Phenylalanin(3-Amidino)-4-(Methylamidobuteryl)Piperidid,
3-(4-Ethoxyphenyl)phenylsulfonyl-L-Phenylalanin(3-Amidino)- 4-(Methylamidobuteryl)Piperidid,
3-(4-Ethylphenyl)phenylsulfonyl-L-Phenylalanin(3-Amidino)- 4-(Methylamidobuteryl)Piperidid,
3-(H-β-Alanyl-NH)phenylsulfonyl-L-Phenylalanin(3-Amidino)-4-Piperidinbuteryl-O-Methylat,
3-(H-β-Alanyl-NH)phenylsulfonyl-L-Phenylalanin(3-Amidino)-4-(Amidobuteryl)Piperidid,
3-(H-β-Alanyl-NH)phenylsulfonyl-L-Phenylalanin(3-Amidino)-4-(Buteryl)Piperidid,
3-(H-β-Alanyl-NH)phenylsulfonyl-L-Phenylalanin(3-Amidino)-Isonipecotylamid,
3-(H-β-Alanyl-NH)phenylsulfonyl-L-Phenylalanin(3-Amidino)-Nipecotylamid,
3-(H-β-Alanyl-NH)phenylsulfonyl-L-Phenylalanin(3-Amidino)-Piperidid,
3-(Pyridazinon)phenylsulfonyl-L-Phenylalanin(3-Amidino)-4-(Methylamidobuteryl) Piperidid,
3-(Piperidinon)phenylsulfonyl-L-Phenylalanin(3-Amidino)-4-(Methylamidobuteryl) Piperidid,
3-(Piperazinon)phenylsulfonyl-L-Phenylalanin(3-Amidino)-4-(Methylamidobuteryl) Piperidid,
3-(4-Ethoxyphenyl)phenylsulfonyl-L-Phenylalanin(3-Hydroxyamidino)-Piperidinbuteryl-N-Methylamid,
3-(4-Ethylphenyl)phenylsulfonyl-L-Phenylalanin(3-Hydroxyamidino)-Piperidinbuteryl-N-Methylamid,
3-(1-Hydroxybutyl)-Phenylsulfonyl-L-Phenylalanin(3-Amidino)-4-(2-Aminoethyl) Piperidid,
3-(3-Azetidin-CONH)-Phenylsulfonyl-L-Phenylalanin(3-Amidino)-4-(Methylamidobuteryl)Piperidid,
3-(3-Azetidin-CONH)-Phenylsulfonyl-L-Phenylalanin(3-Hydroxyamidino)-4-(Methylamidobuteryl)Piperidid,
3-(3-Azetidin-CONH)-Phenylsulfonyl-L-Phenylalanin(3-Amidino)-4-(2-Aminoethyl)Piperidid,
3-(4-tert.Butylphenyl)phenylsulfonyl-L-Phenylalanin(3-Amidino)-4-(Methylamidobuteryl)Piperidid,
3-(4-tert.Butylphenyl)phenylsulfonyl-L-Phenylalanin(3-Hydroxyamidino)-4-(Methylamidobuteryl)Piperidid,
3-(4-tert.Butylphenyl)phenylsulfonyl-L-Phenylalanin(3-Methoxyamidino)-4-(Methylamidobuteryl)Piperidid,
3-(4-Isopropylphenyl)phenylsulfonyl-L-Phenylalanin(3-Amidino)-4-(Methylamidobuteryl)Piperidid,
3-(4-Methoxy-3-pyridyl)phenylsulfonyl-L-Phenylalanin(3-Amidino)-4-(Methylamidobuteryl)Piperidid,
3-(4-Amino-3-Pyridyl)phenylsulfonyl-L-Phenylalanin(3-Amidino)-4-(Methylamidobuteryl)Piperidid,
3-(4-Amino-3-Tetrahydropyridyl)phenylsulfonyl-L-Phenylalanin(3-Amidino)-4-(Methylamidobuteryl)Piperidid,
3-(Na-(CH₃-PEG₁₀₀₀-CH₂-CH₂-CO)-α,β-Diaminopropionyl-NH)phenylsulfonyl-L-Phenylalanin(3-Amidino)-4-(2-Aminoethyl)-Piperidid,
3-(H-β-Alanyl-NH)phenylsulfonyl-L-Phenylalanin(3-Amidino)-4-(CH₃-PEG₁₀₀₀₀-CH₂-CH₂-anlidobuteryl)Piperidid,
3-(CH₃-PEG₁₀₀₀₀-CH₂-CH₂-CO)-β-Ala-NH)phenylsulfonyl-L-Phenylalanin(3-Amidino)-4-(2-Aminoethyl)-Piperidid,
3-(H-β-Alanyl-NH)phenylsulfonyl-L-Phenylalanin(3-Amidino)-Nipecotyl-benzylester.

Ebenfalls bevorzugt ist eine Verbindung gemäß Formel (II), wobei die Verbindung ausgewählt ist aus einer der folgenden Strukturen:
3-(H-βAlanin-NH)phenylsulfonyl-L-Phenylalanin(3-Aminomethyl)-4-(2-Aminoethyl) Piperidid,
3-(4-Methoxyphenyl)phenylsulfonyl-L-Phenylalanin(3-Aminomethyl)-4-(2-Aminoethyl)Piperidid.

Des Weiteren ist eine Verbindung gemäß Formel (III) bevorzugt, wobei die Verbindung ausgewählt ist aus einer der folgenden Strukturen:
3-(H-β-Alanyl-NH)phenylsulfonyl-L-Phenylalanin(3-Amidino)-4-(Ethyloxycarbonyl) Piperazid,
3-(H-β-Alanyl-NH)phenylsulfonyl-L-Phenylalanin(3-Amidino)-4-Benzyloxycarbonyl) Piperazid,
3-(H-β-Alanyl-NH)phenylsulfonyl-L-Phenylalanin(3-Amidino)-Piperazid,
3-(H-β-Alanyl-NH)phenylsulfonyl-L-Phenylalanin(3-Aminomethyl)-4-(Benzyloxycarbonyl)Piperazid,
3-(H-β-Alanyl-NH)phenylsulfonyl-L-Phenylalanin(3-Amidino)-4-(CH₃-PEG₁₀₀₀₀-CH₂-CH₂-NH-Suc)Piperazid.

Des Weiteren sind orale Applikationsformen unter anderem ungeladene oder schwach basische Verbindungen vorteilhaft, da diese besser oral verfügbar sind als die entsprechenden stärker geladenen Verbindungen. Verbindungen mit einer Benzamidingruppe werden deshalb beispielsweise in Form ihrer Prodrugs eingesezt, die erst im Körper in eine Amidinogruppe umgewandelt werden. Geeignete Benzamidin-Prodrugs sind verbindungen mit einer Hydroxyamidino-, Acylamidino-, Alkyloxycarbonylamidino- oder Alkoxyamidinogruppe.

Die entsprechenden Salze der Verbindungen gemäß Formel (I), (II) oder (III) lassen sich leicht mit Hilfe entsprechender organischer oder anorganischer Säuren gewinnen, beispielsweise mit Trifluoressigsäure, Salzsäure, Bernsteinsäure, Essigsäure oder Fumarsäure.

Die erfindungsgemäßen Verbindungen gemäß Formel (I), bei denen R2 ein Aminoalkylrest ist, lassen sich allgemein nach einem Verfahren herstellen, bei dem beispielsweise ein gegebenenfalls geschütztes oder ungeschütztes substituiertes Piperidid, wie z. B. ein 3-(Halogen, vorzugsweise Brom, oder Amino)-Phenylsulfonyl-3-Cyanophenylalanyl-4-(2-geschütztes-amidoalkyl)piperidid an der meta-Position des Phenylrestes mit einem gegebenenfalls geschütztem Rest R1 derivatisiert und anschließend die Cyanogruppe mit Hydroxylamin zum Hydroxylamidin umgesetzt wird, das dann zum Amidin hydriert wird. Die Hydrierung kann entweder direkt oder nach Umsetzung mit z. B. Essigäureanhydrid erfolgen. Am Ende der Synthese werden gegebenenfalls eine oder eventuell mehrere vorhandene Schutzgruppen abgespalten. Beispielsweise kann auch von einem 3-(Halogen, vorzugsweise Brom, oder Amino)-Phenylsulfonyl-3-Cyanophenylalanin ausgegangen werden, welches mit einem geschütztem Amidoalkylpiperidid umgesetzt wird. Entsprechende Verfahrensalternativen können für den Fachmann leicht von den Beispielen abgeleitet werden.

Die erfindungsgemäßen Verbindungen gemäß Formel (I), bei denen R2 ein nicht basischer Rest ist, lassen sich allgemein nach einem Verfahren herstellen, bei dem ein mit einem Halogen, einer Nitro- oder einer Aminogruppe in 3-Position substituiertes Phenylsulfonyl-3-cyanophenylalanin mit einem substituierten Piperidinderivat, z.B. N-Methyl-4-(piperidin-4-yl)butanamid, umgesetzt wird, danach die eventuell vorhandene Nitro- in eine Aminogruppe reduziert wird und dann ein geeigneter Rest R1 direkt oder in gegebenenfalls geschützter Form an den in 3-Position halogen- oder aminosubstituierten Aromaten gekoppelt wird. Anschließend wird, wie oben bereits beschrieben, die Amidinogruppe aufgebaut. Am Ende der Reaktionskette können gegebenenfalls noch vorhandene Schutzgruppen abgespalten werden. Entsprechende Verfahrensalternativen können für den Fachmann wiederum leicht von den Beispielen abgeleitet werden.

Die erfindungsgemäßen Verbindungen gemäß Formel (II) lassen sich allgemein nach einem Verfahren herstellen, bei dem ein geschütztes oder ungeschütztes Arylsulfonyl-1-(3-amidinophenylalanin oder an der Aminogruppe geschütztes Arylsulfonyl-3-aminoalkylphenylalanin, insbesondere ein geschütztes Arylsulfonyl-3-aminomethylphenylalanin, mit einem substituierten Pipderidin umgesetzt und entsprechend derivatisiert wird. Auch in diesem Fall können entsprechende Verfahrensalternativen für den Fachmann leicht von den Beispielen abgeleitet werden.

Die erfindungsgemäßen Verbindungen gemäß Formel (III) lassen sich allgemein nach einem Verfahren herstellen, bei dem ein geschütztes oder ungeschütztes Arylsulfonyl-1-(amidino oder an der Aminogruppe geschütztes 3-alkylamino)phenylalanin, insbesondere ein geschütztes Arylsulfonyl-3-aminomethylphenylalanin, mit einem geschützten bzw. substituierten Piperazin umgesetzt und entsprechend derivatisiert wird. Entsprechende Verfahrensalternativen können für den Fachmann wiederum leicht von den Beispielen abgeleitet werden.

Die Schutzgruppen können jeweils eine tertiäre Butyloxycarbonylgruppe oder eine Benzyloxycarbonylgruppe sein.

Ein weiterer Gegenstand der vorliegenden Erfindung ist ein Arzneimittel enthaltend mindestens eine der erfindungsgemäßen Verbindungen. Das Arzneimittel kann beispielsweise in Form einer Tablette, eines Dragees, einer Kapsel, eines Pellets, eines Suppositoriums, einer Lösung, wie einer Injektions- oder Infusionslösung, von Augen-, Nasen- oder Ohrentropfen, eines Saftes, einer Emulsion oder Suspension, eines Globuli, eines Styli, eines Aerosols, eines Puders, einer Paste, einer Creme oder einer Salbe eingesetzt werden.

Vorteilhafterweise eignen sich die erfindungsgemäßen Arzneimittel bzw. die erfindungsgemäßen Verbindungen zur Therapie oder Prophylaxe eines Tumors, beispielsweise für die orale, subkutane, intravenöse oder transdermale Anwendung. Unter anderem kann hierdurch eine Reduktion der Bildung von Tumormetastasen erreicht werden. Bevorzugt wird daher das erfindungsgemäße Arzneimittel zur Reduktion der Bildung von Tumormetastasen eingesetzt. Eine weitere allgemeine Anwendung der erfindungsgemäßen Arzneimittel und Verbindungen ist die Hemmung der Matriptase. Aus diesem Grund eignet sich die vorliegende Erfindung auch für die in vitro Verwendung einer erfindungsgemäßen Verbindung zur Hemmung der Matriptase.

Die Menge der erfindungsgemäßen Verbindung, die erforderlich ist, um den gewünschten biologischen Effekt zu erreichen, ist abhängig von einer Reihe von Faktoren, z. B. der gewählten spezifischen Verbindung, der beabsichtigten Verwendung, der Art der Verabreichung und dem klinischen Zustand des Patienten.

Im Allgemeinen liegt die Tagesdosis im Bereich von 0,05 mg bis 50 mg pro Tag und pro Kilogramm Körpergewicht (typischerweise von 0,5 mg bis 20 mg pro Tag und pro Kilogramm Körpergewicht), z. B. 1-10 mg pro Tag und Kilogramm Körpergewicht. Eine intravenöse Dosis kann z. B. im Bereich von 0,05 mg bis 10,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 mg pro Milliliter, enthalten. Einzeldosen können z. B. von 1 mg bis 5 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, beispielsweise von 1,0 bis 1000 mg, typischerweise von 10 bis 600 mg enthalten. Im Falle pharmazeutisch verträglicher Salze beziehen sich die vorgenannten Gewichtsangaben auf das Gewicht der dem Salz zugrunde liegenden freien Verbindung. Zur Prophylaxe oder Therapie der oben genannten Zustände können die erfindungsgemäßen Verbindungen selbst verwendet werden, vorteilhafterweise liegen sie jedoch mit einem verträglichen Träger oder Hilfsstoff in Form einer pharmazeutischen Zusammensetzung vor. Der Träger bzw. Hilfsstoff muss natürlich verträglich sein, in dem Sinn, dass er mit den anderen Bestandteilen der Zusammensetzung kompatibel ist und nicht gesundheitsschädlich für den Patienten ist.

Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew-% des Wirkstoffs enthalten kann. Weitere pharmazeutisch aktive Substanzen können ebenfalls vorhanden sein, einschließlich weiterer erfindungsgemäßer Verbindungen. Die erfindungsgemäßen Arzneimittel können nach einer der bekannten pharmazeutischen Methoden hergestellt werden, die im Wesentlichen darin bestehen, dass die Bestandteile mit pharmakologisch verträglichen Träger- und/oder Hilfsstoffen gemischt werden.

Vorteilhafte pharmazeutische Zusammensetzungen sind unter anderem solche, die für orale, rektale, topische, perorale (z. B. sublinguale) und parentale (z. B. subkutane, intramuskuläre, intradermale oder intravenöse) Verabreichung geeignet sind, wenngleich die am besten geeignete Verabreichungsweise in jedem Einzelfall von der Art und Schwere des zu behandelnden Zustandes und von der Art der jeweils verwendeten erfindungsgemäßen Verbindung abhängig ist. Auch dragierte Formulierungen und dragierte Retard-Formulierungen gehören in den Rahmen der Erfindung. Bevorzugt sind säure- und magensaftresistente Formulierungen. Geeignete magensaftresistente Beschichtungen umfassen Celluloseacetatphthalat, Polyvinylacetatphthalat, Hydroxypropylmethylcellulosephthalat und anionische Polymere von Methacrylsäure und Methacrylsäuremethylester.

Geeignete pharmazeutische Verbindungen für die orale Verabreichung, die besonders günstig sind, können in separaten Einheiten vorliegen, wie zum Beispiel Kapseln, Oblatenkapseln, Lutschtabletten oder Tabletten, die jeweils eine bestimmte Menge der erfindungsgemäßen Verbindung enthalten; als Pulver oder Granulate; als Lösung oder Suspension in einer wässrigen oder nicht-wässrigen Flüssigkeit; oder als eine Öl-in-Wasser- oder Wasser-in-Öl-Emulsion. Diese Zusammensetzungen können, wie bereits erwähnt, nach jeder geeigneten pharmazeutischen Methode zubereitet werden, die einen Schritt umfasst, bei dem der Wirkstoff und der Träger (der aus einem oder mehreren zusätzlichen Bestandteilen bestehen kann) in Kontakt gebracht werden. Im Allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird.

So kann beispielsweise eine Tablette hergestellt werden, indem ein Pulver oder Granulat der Verbindung verpresst oder geformt wird, gegebenenfalls mit einem oder mehreren zusätzlichen Bestandteilen. Gepresste Tabletten können durch Tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine erfindungsgemäße Verbindung mit einem Geschmacksstoff enthalten, üblicherweise Saccharose oder Gummi arabicum oder Tragant, und Pastillen, die die Verabreichung einer inerten Basis wie Gelatine und Glycerin oder Saccharose und Gummi arabicum umfassen.

Geeignete pharmazeutische Zusammensetzungen für die parentale Verabreichung umfassen beispielsweise sterile wässrige Zubereitungen einer erfindungsgemäßen Verbindung, die vorzugsweise isotonisch mit dem Blut des vorgesehenen Empfängers sind. Diese Zubereitungen werden unter anderem intravenös verabreicht, wenngleich eine Verabreichung auch subkutan, intramuskulär oder intradermal als Injektion erfolgen kann. Diese Zubereitungen können günstigerweise hergestellt werden, indem die Verbindung mit Wasser gemischt wird und die erhaltene Lösung steril und mit dem Blut isotonisch gemacht wird. Injizierbare erfindungsgemäße Arzneimittel enthalten im Allgemeinen von 0,1 bis 5 Gew.-% der aktiven Verbindung. Bezüglich weiterer Formulierung wird auf gängige Handbücher verwiesen.

Offenbart ist somit auch ein Verfahren zur Herstellung eines Arzneimittels, bei denen eine oder mehrere erfindungsgemäße Verbindungen mit geeigneten Träger- und Hilfsstoffen, wie oben beschrieben, vermischt werden.

Die folgenden Verfahren und Beispiele dienen zur näheren Erläuterung der Erfindung, ohne sie zu beschränken.

### Verfahren zur Analyse der erfindungsgemäßen Verbindungen

### Analytische HPLC

Zur analytischen reversed-phase-HPLC wurde eine HPLC-Anlage LC-10A der Firma Shimadzu, bestehend aus den Teilsystemen CTO-10AS Säulenofen, LC-10AD Pumpen (2 x), DGU-14A Degaser, SIL-10AD Autoinjektor, SCL-10A Systemcontroller, SPD-10A UV-Vis Detektor und einer Säule Luna 5 µm C18(2) 100 Å, 250 x 4,6 mm der Firma Phenomenex, unter Benutzung der zugehörigen Software Shimadzu CLASS-VP, Version 5.3, verwendet. Die Detektion erfolgte bei 220 nm. Als Elutionsmittel dienten Wasser mit 0,1 % TFA (A) und Acetonitril mit 0,1 % TFA (B) bei einer Flussrate von 1 ml/min und einem linearen Gradienten (1 % B/min). Zur Analyse sämtlicher Polyethylenglykolmodifizierter Wirkstoffe wurde eine Jupiter-Säule 5 µm C18(2) 300 Å, 250 x 4,6 mm der Firma Phenomenex verwendet.

### Präparative HPLC

Zur präparativen RP-HPLC wurde eine HPLC-Anlage der Firma Shimadzu, bestehend aus den Teilsystemen LC-8A präparative Pumpen (2 x), DGU-14A Degaser, FRC-10A Fraktionssammler, SCL-10A Systemcontroller, SPD-10A UV-Vis Detektor und einer Säule Luna 5 µm C8(2) 100 Å, 250 x 30,0 mm der Firma Phenomenex, unter Benutzung der zugehörigen Software Shimadzu CLASS-VP, Version 5.3, verwendet. Die Detektion erfolgte bei 220 nm. Als Elutionsmittel dienten ebenfalls Wasser mit 0,1 % TFA (A) und Acetonitril mit 0,1 % TFA (B) bei einer Flussrate von 10 oder 20 ml/min und einem geeigneten Gradienten.

### Massenspektroskopie

Die Massenspektren wurden standardmäßig auf einem ESI-MS LCQ der Firma Finnigan (Bremen, Deutschland), gemessen. Sämtliche Polyethylenglykol-gekoppelten Verbindungen wurden auf einem Maldi Ultraflex Tof/Tof Gerät der Firma Bruker analysiert.

### Dünnschichtchromatographie

Für die Dünnschichtchromatographie wurden Kieselgel-Fertigplatten Adamant UV₂₅₄ der Firma Macherey-Nagel verwendet. Als Laufmittel diente ein Gemisch aus n-Butanol, Eisessig und Wasser (4:1:1). Die Detektion der Verbindungen erfolgte durch UV-Absorption bei 254 nm, außerdem wurden als Sprühreagenzien eine Ninhydrinlösung (300 mg Ninhydrin gelöst in 100 ml *n*-Butanöl und 3 ml Eisessig) und nach Inkubation der DC-Platte in Chloratmosphäre eine *o*-Tolidinlösung (150 mg o-Tolidin und 2,1 g KI gelöst in 2 ml Eisessig und 148 ml Wasser) verwendet.

### Verwendete Abkürzungen

- Ac: Acetyl
- ACN: Acetonitril
- Ame: Aminomethyl
- Boc: tert.-Butyloxycarbonyl
- Bz: Benzoyl
- Bzl: Benzyl
- Bzls: Benzylsulfonyl
- Cbz: Benzyloxycarbonyl
- CKIBE: Chlorkohlensäureisobutylester
- Dap: α,β-Diaminopropionsäure
- DIEA: Diisopropylethylamin
- DCM: Dichlormethan
- DMF: N,N-Dimethylformamid
- HPLC: Hochleistungs-Flüssigkeitschromatographie
- iNip: Isonipecotinsäure
- iPr: *iso*-Propyl
- i.V.: im Vakuum
- LM: Lösungsmittel
- MS: Massenspektroskopie
- n.b.: nicht bestimmt
- Nip: Nipecotinsäure
- NMM: N-Methylmorpholin
- PEG: Polyethylenglykol
- PyBop: Benzotriazol-1-yl-N-oxy-tris(pyrrolidino)phosphoniumhexafluorophosphat
- Pzd: Piperazid
- RT: Raumtemperatur
- Suc: Succinyl
- tBu: tert.-Butyl
- TEA: Triethylamin
- Tfa: Trifluoracetyl
- TFA: Trifluoressigsäure

### Beispiele

### Beispiel 1:

### 3-(3,4-Dimethoxyphenyl)phenylsulfonyl-d/l-Phe(3-Am)-4-(2-Aminoethyl)Piperidid × 2 TFA

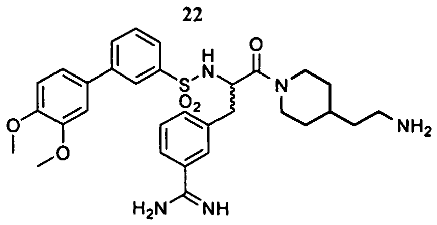

### 1a) 3-Bromphenylsulfonyl-d/l-Phe(3-CN)-OH

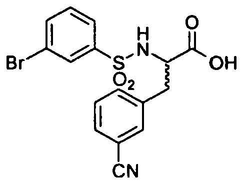

1 g (5,26 mmol) H-d/1-Phe(3-CN)-OH (Senn Chemicals AG, Dielsdorf, Schweiz) wird in 20 ml Wasser und 4 ml Dioxan suspendiert und mit 1,006 ml (5,78 mmol) DIEA versetzt. Unter Eiskühlung werden 1,478 g (5,78 mmol) 3-Bromphenyl-Sulfonylchlorid (Alfa Aesar), gelöst in 16 ml Dioxan, innerhalb von 30 min zugetropft. Der pH wird kontrolliert, und durch zusätzliche Gabe von DIEA auf pH 8-9 eingestellt. Es wird eine weitere Stunde bei 0 °C und über Nacht bei RT gerührt. Das Lösungsmittel wird im Vakuum entfernt und der Rückstand in Wasser unter Zugabe von 1 N NaOH (pH ca. 10) gelöst. Die Wasserphase wird 1 x mit Essigester extrahiert und mit 6 N HCl angesäuert (pH ca. 2). Die Wasserphase wird 3 x mit Essigester extrahiert. Anschließend wird der Essigester 3 x mit gesättigter NaCl-Lösung gewaschen, das LM mit Na₂SO₄ getrocknet und im Vakuum entfernt.
Ausbeute: 1.9 g (4,64 mmol) gelblicher Feststoff
HPLC: 49,2 % B

### 1b) 3-Bromphenylsulfonyl-d/l-Phe(3-CN)-4-(2-Boc-amidoethyl)Piperidid

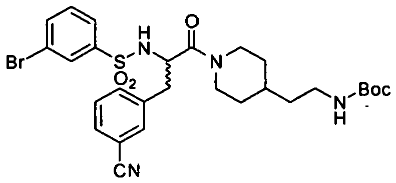

1,5 g (3,66 mmol) 3-Bromphenylsulfonyl-d/1-Phe(3-CN)-OH und 837 mg (3,66 mmol) 4-(2-Boc-amidoethyl)Piperidid (Steinmetzer et al., J. Med. Chem. 49, 2006, 4116) werden in 10 ml DMF unter Zugabe von 1,27 ml (7,32 mmol) DIEA gelöst und bei 0 °C mit 1,9 g (3,66 mmol) PyBop versetzt. Der Ansatz wird 30 min bei 0 °C und 3 h bei RT gerührt und das LM im Vakuum entfernt. Der Rückstand wird in EE aufgenommen und 3 x mit 5 % KHSO₄-Lösung, 1 x mit gesättigter NaCl-Lösung, 3 x mit gesättigter NaO₃-Lösung und 3 x mit gesättigter NaCl-Lösung gewaschen. Das LM wird mit Na₂SO₄ getrocknet und im Vakuum entfernt. Der verbleibende Rückstand wird mittels präparativer HPLC gereinigt und das Lösungsmittel i.V. entfernt.
Ausbeute: 1,35 g Öl
HPLC: 64,16 % B

### 1c) 3-(3,4-Dimethoxyphenyl)phenylsulfonyl-d/l-Phe(3-CN)-4-(2-Boc-amidoethyl)-Piperidid

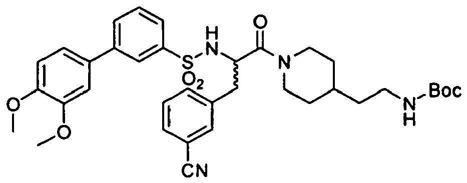

67,1 mg (0,108 mmol) 3-Bromphenylsulfonyl-d/1-Phe(3-CN)-4-(2-Bocamidoethyl)Piperidid und 29,6 mg 3,4-Dimethoxyphenylboronsäure (Acros) werden mit 3 ml Toluol und 200 µl einer 2 M Cs₂CO₃-Lösung versetzt. Nach Zugabe von ca. 1 mol% Pd-(II)-Acetat und ca. 2 mol% 2-Dicyclohexylphosphin-2',6'-dimethoxybiphenyl (S-Phos, Aldrich) wird der Ansatz unter Argon und 3 h unter Rückfluß erhitzt. Der Ansatz wird zentrifugiert und das LM des Überstandes i.V. entfernt. Der Rückstand wird in Essigester gelöst und 2 x mit halbgesättigter NaCl-Lösung gewaschen. Der Essigester wird mit Na₂SO₄ getrocknet und i.V. entfernt.
Ausbeute: 40 mg Öl
HPLC: 64,39 % B, MS ber.: 676,29; gef.: 577,3 (M+H)⁺ nach Boc-Abspaltung mit TFA

### 1d) 3-(3,4-Dimethoxyphenyl)phenylsulfonyl-d/l-Phe(3-Acetylhydroxyamidin)-4-(2-Bocamidoethyl)Piperidid

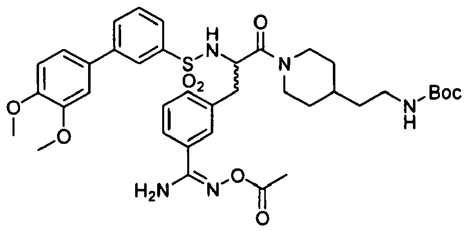

40 mg des Rohproduktes an 3-(3,4-Dimethoxyphenyl)phenylsulfonyl-d/1-Phe(3-CN)-4-(2-Boc-amidoethyl)Piperidid werden in 1,5 ml absolutem Ethanol gelöst und mit 12,2 mg (0,175 mmol) Hydroxylamin x HCl und 30,4 µl (0.175 mmol) DIEA versetzt. Der Ansatz wird 4 h unter Rückfluß und über Nacht bei RT gerührt (HPLC: 48,9 %B). Das LM wird i.V. entfernt, der Rückstand in 1 ml Essigsäure gelöst und mit 15 µl Essigsäureanhydrid versetzt. Nach 30 min wird das LM i.V. entfernt (Öl, enthält Salze).
HPLC: 58,37 % B

### 1e) 3-(3,4-Dimethoxyphenyl)phenylsulfonyl-d/1-Phe(3-Am)-4-(Aminoethyl)Piperidid x 2 TFA

Das Rohprodukt 1d wird in 5 ml 90 % Essigsäure gelöst und mit Wasserstoff und ca. 5 mg 10 % Palladium auf Aktivkohle als Katalysator über Nacht hydriert. Der Katalysator wird abfiltriert, das LM i.V. entfernt und der Rückstand (HPLC: 48,83 % B) mit 1,5 ml TFA versetzt. Nach 1 h wird das LM i.V. entfernt und der Rückstand mit präparativer HPLC gereinigt.
Ausbeute: 17,5 mg weißes lyophilisiertes Pulver
HPLC: 31,48 % B, MS ber.: 593,27 gef.: 594,3 (M+H)⁺

### Beispiele 2-15:

Auf analoge Weise, unter Verwendung der entsprechenden kommerziell erhältlichen Boronsäuren bzw. Boronsäure-Pinacolester, wurden weitere Inhibitoren synthetisiert (Tabelle 1):

**Tabelle 1:**

| Beispiel/Nr. | Struktur | HPLC (% B) | MS berechnet gefunden (M+H)⁺ |
|---|---|---|---|
| 2 | 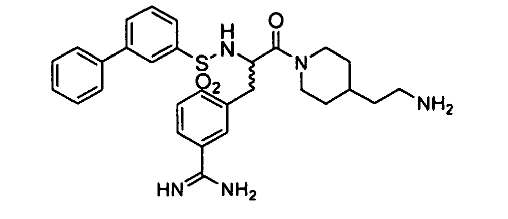 | 31,92 | 533,25 534,4 |
| 3 | 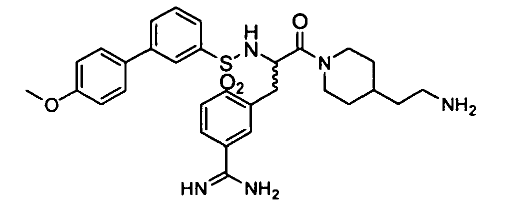 | 32,36 | 563,26 564,3 |
| 4 | 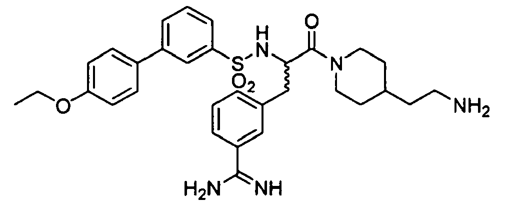 | 35,94 | 577,27 578,4 |
| 5 | 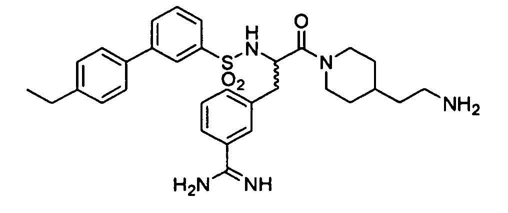 | 37,89 | 561,28 562,3 |
| 6 | 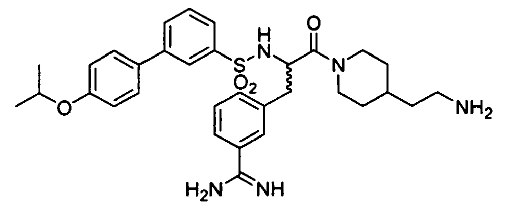 | 38,25 | 591,29 592,3 |
| 7^{a} | 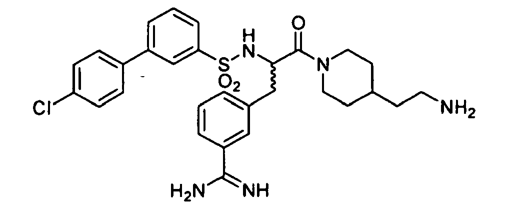 | 35,46 | 567,21 568,3 |
| 8^{a} | 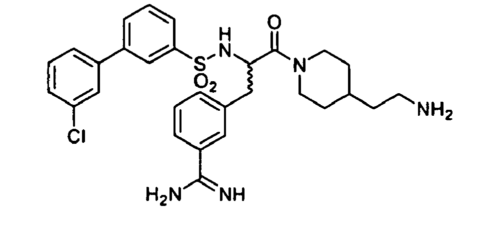 | 35,82 | 567,21 568,3 |
| 9^{a} | 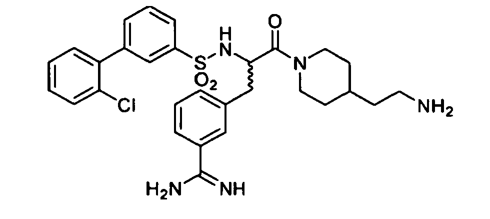 | 34,88 | 567,21 568,4 |
| 10^{b} | 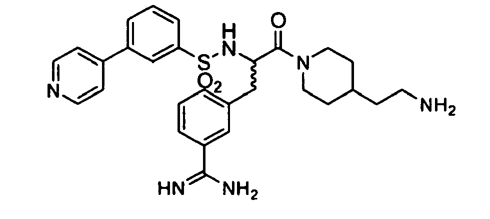 | 19,3 | 534,24 535,3 |
| 11^{b} | 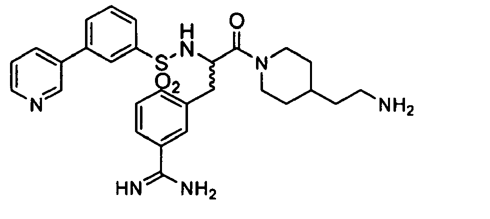 | 19,1 | 534,24 535,6 |
| 12^{c} | 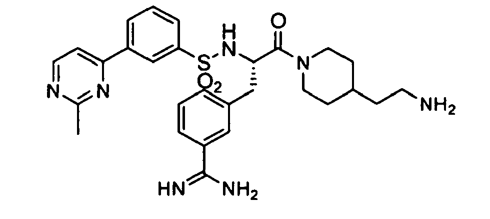 | 24,16 | 549,25 550,3 |
| 13 | 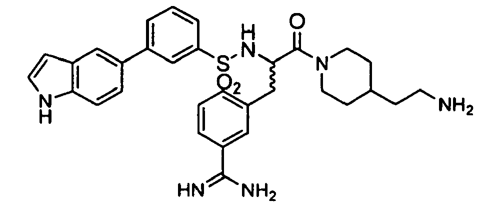 | 32,62 | 572,26 573,3 |
| 14^{b,d} | 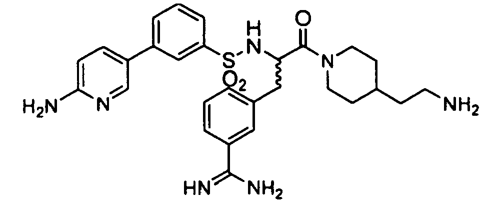 | 20,4 | 549,25 550,3 |
| 15^{e} | 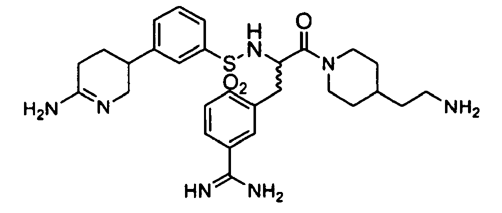 | 19,6 | 553,28 554,4 |

| | | | |
|---|---|---|---|
| ^{a}Die Amidinogruppe der Inhibitoren 7-9 wurde durch Reduktion des Acetylhydroxyamidins durch Zink in Eisessig aufgebaut (Steinmetzer et al., J. Med. Chem. 49, 2006, 4116). ^{b}Der Suzuki-Kupplungsschritt c für die Synthese dieser Inhibitoren wurde in einem fokussierten Mikrowellen-Synthesesystem "Discover" der Firma CEM (t = 60-120 min, T = 100-120°C, 200-240 W) durchgeführt. ^{c}Für die Synthese dieser Verbindung wurde kommerziell erhältliches 3-(2-Methyl-4-Pyrimidylsulfonyl-Cl (Maybridge) direkt an 3-1-Cyanophenylalanin gekoppelt. ^{d}Für die Synthese dieser Verbindung wurde 6-Aminopyridin-3-boronsäure-Pinacolester (Aldrich) für den analogen Schritt c (siehe Beispiel 1) eingesetzt, wobei dass erhaltene Zwischenprodukt mit Boc-Pyrocarbonat umgesetzt wurde. Die folgenden Reaktionsschritte erfolgten analog der Synthese von Inhibitor 1 ^{e}Die Verbindung wurde durch Hydrierung des Inhibitors 14 mit Pd/C als Katalysator unter Standardbedingungen (Wasserstoff, 1 bar) erhalten | | | |

### Beispiel 16:

### 3-(1-Aminobutyl)-Phenylsulfonyl-Phe(3-Amidino)-4-(2-Aminoethyl)Piperidid x 3 TFA

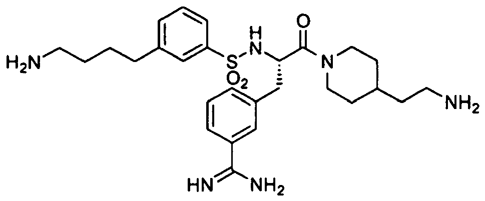

### 16a) 1-(Cbz-Amino)-3-Buten

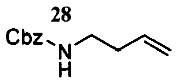

500 mg (7,03 mmol) 3-Buten-1-Amin (Aldrich) werden in 15 ml Dioxan suspendiert und mit 7,1 ml 1 N NaOH versetzt. Die klare Lösung wird im Eisbad gekühlt und portionsweise mit insagesamt 990 µl Cbz-Cl versetzt. Der pH wird durch Zugabe von weiterer NaOH-Lösung auf 9-10 eingestellt. Der Ansatz wird eine Stunde bei 0 °C und über Nacht bei RT gerührt. Das LM wird i.V. entfernt, der Rückstand in Essigester aufgenommen und 2 x mit gesättiger NaCl-Lösung gewaschen. Das Lösungsmittel wird mit Na₂SO₄ getrocknet und i.V. entfernt.
Ausbeute: 980 mg farbloses Öl
DC: (4/1/1 n-Butanol/Eisessig/Wasser): R_{f}= 0,86

### 16b) 3-Iodphenylsulfonyl-Phe(3-CN)-OH

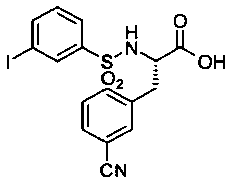

0,76 g (4 mmol) H-Phe(3-CN)-OH (Senn Chemicals AG, Dielsdorf, Schweiz) werden in 20 ml Wasser und 4 ml Dioxan suspendiert und unter Zugabe von 765 µl (4,4 mmol) DIEA gelöst. Unter Eiskühlung wird eine Lösung aus 1,33 g (4,4 mmol) 3-Iodphenyl-Sulfonylchlorid (hergestellt analog Langmuir, Chem. Berichte 28, 90-96, 1895) gelöst in ca. 15 ml Dioxan, innerhalb von 30 min zugetropft. Der pH Wert wird kontrolliert und durch Zugabe von DIEA (insgesamt 830 µl in mehreren Portionen) auf 8-9 eingestellt. Es wird weitere 30 min bei 0 °C und 4 h bei RT gerührt. Das Lösungsmittel wird im Vakuum entfernt und der Rückstand in Wasser unter Zugabe von 1 N NaOH (pH ca. 10) gelöst. Die Wasserphase wird 1x mit Essigester extrahiert und mit 6 N HCl angesäuert (pH ca. 2). Die Wasserphase wird 3 x mit Essigester extrahiert. Anschließend wird der Essigester 3 x mit gesättigter NaCl-Lösung gewaschen, das LM mit Na₂SO₄ getrocknet und im Vakuum entfernt.
Ausbeute: 1.7 g (3,72 mmol) schwach gelblicher Feststoff
HPLC: 50,28 % B

### 16c) 3-Iodphenylsulfonyl-Phe(3-CN)-4-(2-Boc-amidoethyl)Piperidid

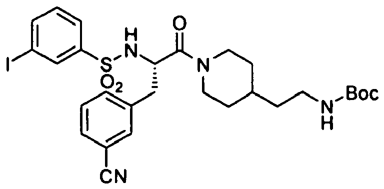

1,14 g (2,5 mmol) 3-Iodphenylsulfonyl-d/1-Phe(3-CN)-OH und 570 mg (2,5 mmol) 4-(2-Boc-amidoethyl)Piperidid (Steinmetzer et al., J. Med. Chem. 49, 2006, 4116) werden in 8 ml DMF unter Zugabe von 0,87 ml (5 mmol) DIEA gelöst und bei 0 °C mit 1,3 g (2,5 mmol) PyBop versetzt. Der Ansatz wird 30 min bei 0 °C und 4 h bei RT gerührt und das LM im Vakuum entfernt. Der Rückstand wird in EE aufgenommen und 3 x mit 5 % KHSO₄-Lösung, 1 x mit gesättigter NaCl-Lösung, 3 x mit gesättigter NaHCO₃-Lösung und 3 x mit gesättigter NaCl-Lösung gewaschen. Das LM wird mit Na₂SO₄ getrocknet und im Vakuum entfernt. Der verbleibende Rückstand wird mittels präparativer HPLC gereinigt und das Lösungsmittel i.V. entfernt..
Ausbeute: 1,1 g lyophilisiertes Pulver
HPLC: 65,17 % B

### 16d) 3-(Cbz-NH-(CH₂)₂-CH=CH)-Phenylsulfonyl-Phe(3-CN)-4-(2-Boc-amidoethyl)-Piperidid

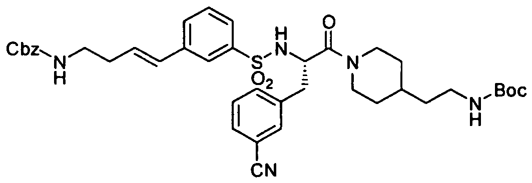

170 mg (0,255 mmol) 3-Iodphenylsulfonyl-d/1-Phe(3-CN)-4-(2-Boc-amidoethyl)Piperidid, 5 mg Pd-(II)-Acetat und 65 mg (0.771 mmol) NaHCO₃ werden mit 3 ml DMF und 2 ml Wasser versetzt. Der Ansatz wird auf 50 ° C erhitzt, mit 68 mg (0,244 mmol) Tetrabutylammonium-Chlorid und 58 mg (0,28 mmol) Cbz-NH-(CH₂)₂-CH=CH₂ versetzt und über Nacht bei 50 °C gerührt. Das Lösungsmittel wird i.V. entfernt, der Rückstand in 50 % Acetonitril gelöst und durch Zugabe von Essigsäure auf pH 4 eingestellt. Das Produkt wird durch präparative HPLC gereinigt und das Lösungsmittel der Produktenthaltenden Fraktionen i.V. entfernt.
Ausbeute: 65 mg Öl, HPLC: 67,44 und 67,92 % im Verhältnis 3:1
MS: ber. 743,34 gef. 766,5 (M+Na)⁺

### 16e) 3-(Cbz-NH-(CH₂)₄)-Phenylsulfonyl-Phe(3-CN)-4-(2-Boc-amidoethyl)Piperidid

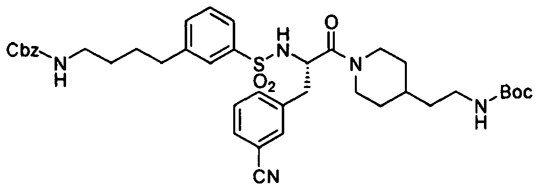

65 mg des öligen 3-(Cbz-NH-(CH₂)₂-CH=CH)-Phenylsulfonyl-Phe(3-CN)-4-(2-Bocamidoethyl)Piperidid werden in 50 ml Essigester gelöst und mit einer geringen Menge an Pd/C-Katalysato versetzt und mit Wasserstoff über Nacht hydriert. Der Katalysator wird abfiltriert und das LM i.V. entfernt.
Ausbeute: 45 mg farbloses Öl,
HPLC: 68,28 %, MS: ber. 745,34 gef. 768,5 (M+Na)⁺

### 16f) 3-(Cbz-NH-(CH₂)₄)-Phenylsulfonyl-Phe(3-Acetyl-Hydroxyamidino)-4-(2-Boc-amido-ethyl)Piperidid

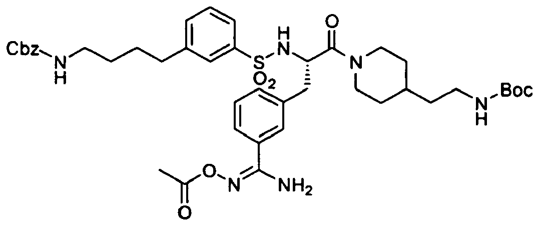

44 mg (ca. 0,06 mmol) 3-(Cbz-NH-(CH₂)₄)-Phenylsulfonyl-Phe(3-CN)-4-(2-Bocamidoethyl)Piperidid werden in 2 ml Ethanol gelöst und mit 17 mg (0,24 mmol) Hydroxylamin x HCl und 42 µl (0,24 mmol) DIEA versetzt. Der Ansatz wird 6 h unter Rückfluß erhitzt und über Nacht bei RT gerührt. Das Lösungsmittel wird i.V. entfernt, der Rückstand (HPLC: 51,78 % B) in 2 ml Essigsäure gelöst und mit 47 µl (0,5 mmol) Essigsäureanhydrid versetzt. Der Ansatz wird 30 min gerührt und das Lösungsmittel i.V. entfernt. Das Rohprodukt (HPLC: 62,6 % B) wird direkt für den nächsten Reaktionsschritt verwendet.

### 16) 3-(1-Aminobutyl)-Phenylsulfonyl-Phe(3-Amidino)-4-(2-Aminoethyl)Piperidid x 3 TFA

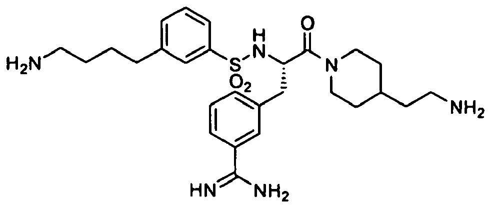

Das Rohprodukt an 3-(Cbz-NH-(CH₂)₄)-Phenylsulfonyl-Phe(3-Acetyl-Hydroxyamidino)-4-(2-Boc-amidoethyl)Piperidid wird in 10 ml 90 % Essigsäure gelöst und über Nacht mit Pd/C als Katalysator und Wasserstoff hydriert. Der Katalysator wird abfiltriert, das Filtrat i.V. eingeengt und der Rückstand (HPLC: 33,76 % B) mit 2 ml TFA versetzt. Der Ansatz wird eine Stunde geschüttelt, das Lösungsmittel i.V. weitestgehend eingeengt und das Produkt mit präparativer HPLC gereinigt und lyophilisiert.
Ausbeute: 15 mg lyophilisiertes Pulver
HPLC: 19,48 %, MS: ber. 528,29 gef. 529.29 (M+H)⁺

### Beispiel 17:

### N-(3-β-Ala)amidophenylsulfonyl-Phe(3-Am)-Piperidinbuteryl-N-Methylamid x 2 TFA

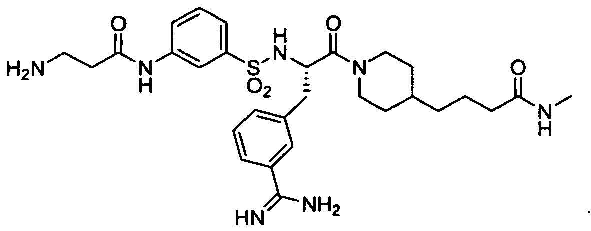

### 17a) H-Phe(3-CN)-OMe × HCl

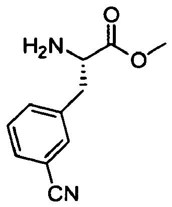

945 mg (4,97 mmol) H-Phe(3-CN)-OH werden in 4 ml Methanol suspendiert und bei -15 °C tropfenweise mit 500 µl (6,87 mmol) Thionylchlorid versetzt. Nachdem ca. 1/3 des Thionylchlorids zugegeben waren, löst sich die Aminosäure, nach vollständiger Zugabe fällt jedoch teilweise Produkt aus. Der Ansatz wird weitere 30 min bei -15°C gerührt und dann langsam auf RT erwärmt. Der Ansatz wird weitere 5 h bei RT gerührt, wobei sich alles löst. Das Lösungsmittel wird i.V. weitestgehend eingeengt und das Produkt durch Zugabe von Ether gefällt, abgesaugt und mit Ether gewaschen.
Ausbeute: 1,19 g (4,94 mmol) weißer Feststoff
HPLC: 20,53 % B

### 17b) 3-NO₂-Phenylsulfonyl-Phe(3-CN)-OMe

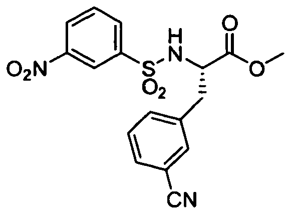

580 mg (2,4 mmol) H-Phe(3-CN)-OMe x HCl werden unter Zugabe von 418 µl (2,4 mmol) DIEA in 2 ml DMF gelöst. Bei 0 °C werden 560 mg (2,52 mmol) 3-NO₂-Phenylsulfonyl-Chlorid in mehreren Portionen zugesetzt, der pH wird mit weiterem DIEA auf pH 8-9 eingestellt. Der Ansatz wird 1 h bei 0 °C und über Nacht bei RT gerührt. Das Lösungsmittel wird i.V. entfernt und der Rückstand in Essigester gelöst, 3 x mit 5 % KHSO₄-Lösung, 1 x mit gesättigter NaCl-Lösung, 3 x mit gesättigter NaHCO₃-Lösung und 3 x mit gesättigter NaCl-Lösung gewaschen. Das LM wird mit Na₂SO₄ getrocknet und i.V. entfernt.
Ausbeute: 580 mg (1,488 mmol) gelbliches Öl
HPLC: 52,45 % B

### 17c) 3-NH₂-Phenylsulfonyl-Phe(3-CN)-OMe

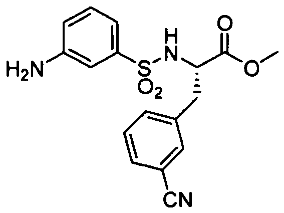

580 mg 3-NO₂-Phenylsulfonyl-Phe(3-CN)-OMe werden in 15 ml 90 % Essigsäure gelöst und mit Zinkstaub versetzt. Der Ansatz wird 2 h bei RT gerührt, der verbleibende Rest an Zinkstaub abfiltriert und das Lösungsmittel i.V. entfernt. Der Rückstand wird in Essigester aufgenommen, 2 x mit gesättigter NaHCO₃-Lösung und 2 x mit gesättigter NaCl-Lösung gewaschen. Das LM wird mit Na₂SO₄ getrocknet und im Vakuum entfernt.
Ausbeute: 405 mg (1,12 mmol) gelbes Öl
HPLC: 39,75 % B

### 17d) Cbz-βAla-3-NH-phenylsulfonyl-Phe(3-CN)-OMe

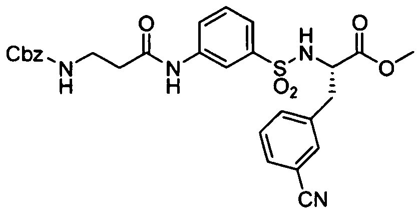

200 mg (0.90 mmol) Cbz-β-Ala-OH werden in 4 ml DMF gelöst, mit 100 µl NMM (0,90 mmol) versetzt und auf -15°C gekühlt. Es werden 117 µl CKIBE (0,90 mmol) zugegeben und der Ansatz weitere 10 min bei -15 °C gerührt. Danach werden 380 mg (1,06 mmol) 3-NH₂-Phenylsulfonyl-Phe(3-CN)-OMe und 50 µl NMM (0,45 mmol) zugesetzt und der Ansatz 1 h bei -15 °C über Nacht bei RT gerührt. Das LM wird i.V. entfernt, der Rückstand in Essigester aufgenommen und 3 x mit 5 % KHSO₄-Lösung, 1 x mit gesättigter NaCl-Lösung, 3 x mit gesättigter NaHCO₃-Lösung und 3 x mit gesättigter NaCl-Lösung gewaschen. Das LM wird mit Na₂SO₄ getrocknet und im Vakuum entfernt.
Ausbeute: 460 mg (0,81 mmol) gelbes Öl
HPLC: 54,15 % B, MS ber.: 564,17 gef.: 563,0 (M-H)⁻

### 17e) Cbz-βAla-3-NH-phenylsulfonyl-Phe(3-CN)-OH

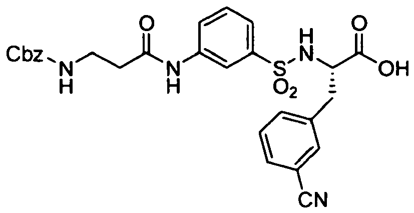

455 mg (0,806 mmol) Cbz-βAla-3-NH-phenylsulfonyl-Phe(3-CN)-OMe werden in 2 ml Dioxan gelöst und mit 2 ml 1 N LiOH versetzt. Die homogene Lösung wird 2 h bei RT gerührt und danach mit 2 ml 1 N HCl neutralisiert. Das LM wird i.V. entfernt, der Rückstand in Essigester aufgenommen und 3 x mit 5 % KHSO₄-Lösung und drei mal mit gesättigter Kochsalzlösung gewaschen. Die organische Phase wird über Na₂SO₄ getrocknet und im Vacuum eingeengt.
Ausbeute: 390 mg Schaum
HPLC: 48,99 % B" MS ber.: 550,15; gef.: 549.0 (M-H)⁻.

### 17f) Cbz-βAla-3-NH-phenylsulfonyl-Phe(3-OxAm)-OH

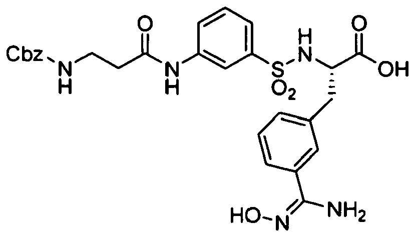

390 mg (0,71 mmol) Cbz-βAla-3-NH-phenylsulfonyl-Phe(3-CN)-OH werden in 5 ml abs. Ethanol gelöst, mit 73 mg (1,05 mmol) Hydroxylaminhydrochlorid und 184 µl (1,05 mmol) DIEA versetzt und 4 h unter Rückfluß gekocht. Es wird über Nacht bei RT weitergerührt. Das Lösungsmittel wird i.V. entfernt und der verbleibende Rückstand mittels präperativer HPLCgereinigt und das Produkt lyophilisiert.
Ausbeute: 202 mg lyophilisiertes Pulver
HPLC: 35,68 % B

### 17g) Cbz-βAla-3-NH-phenylsulfonyl-Phe(3-AcOxAm)-OH

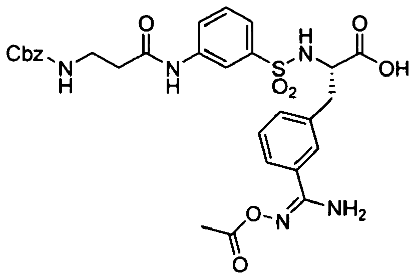

202 mg Cbz-βAla-3-NH-phenylsulfonyl-Phe(3-OxAm)-OH werden in 5 mL Eisessig gelöst, mit 143 µl (1,5 mmol) Acetanhydrid versetzt und eine Stunde bei Raumtemperatur gerührt. Das Lösungsmittel wird im Vakuum entfernt und der Rückstand aus *tert* Butanol lyophilisiert.
Ausbeute: 240 mg lyophilisiertes Pulver
HPLC: 44,95 % B

### 17h) Cbz-4-Piperidinbuttersäure

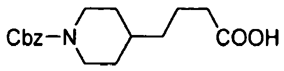

3,984 g (19,18 mmol) 4-Piperidinbuttersäure × HCl werden in 15 ml Wasser und 40 ml 1 N NaOH gelöst und unter Eiskühlung tropfenweise mit 3,3 ml (23,2 mmol) Cbz-Cl, gelöst in 5 ml Dioxan, versetzt. Der pH-Wert wird kontrolliert und auf pH 9-10 konstant gehalten. Der Ansatz wird eine weitere Stunde bei 0 °C und über Nacht bei RT gerührt. Das LM wird i.V. entfernt, der Rückstand in basischem Wasser gelöst und 2 × mit Essigester extrahiert. Die wässrige Phase wird durch Zugabe von HCl angesäuert und 3 × mit Essigester extrahiert. Die Essigesterphase wird 3 × mit gesättigter NaCl-Lösung gewaschen, mit Na₂SO₄ getrocknet und das LM i.V. eingeengt.
Ausbeute: 5,6 g farbloses Öl, MS ber.: 305,16; gef.: 304,2 (M-H)⁻.
HPLC: 51,84 % B

### 17i) Cbz-4-Piperidinbuteryl-N-Methylamid

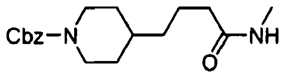

570 mg (ca. 1.86 mmol) Cbz-4-Piperidinbuttersäure wurden in 7 ml DMF gelöst und bei 0 °C mit 135 mg (2 mmol) Methylamin ×HCl, 968 mg (1,86 mmol) PyBop und 647 µl (3,72 mmol) DIEA versetzt. Der Ansatz wurde 30 min unter Eiskühlung und weitere 3 h bei RT gerührt. Das LM wird i.V. entfernt und das Produkt durch präparative HPLC gereinigt und aus 80 % *tert*.Butanol lyophilisiert.
Ausbeute: 230 g farbloses Öl, MS ber.: 318,19; gef.: 319,2 (M+H)⁺.
HPLC: 47,51 % B

### 17j) H-Piperidinbuteryl-N-Methylamid × HBr

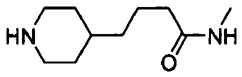

150 mg des öligen Cbz-4-Piperidinbuteryl-N-Methylamid werden mit 1 ml 35 % HBr in Eisessig versetzt. Der Ansatz wird 1 h bei RT stehen gelassen und danach mit Ether versetzt. Das Produkt scheidet sich am Kolbenrand in öliger Form ab, der Überstand wird abdekantiert, der Rückstand in Methanol gelöst und nochmals mit Ether gefällt. Der Überstand wird wieder abdenkantiert und der Rückstand im Vakuum getrocknet.
Ausbeute: 129 mg weißer Feststoff

### 17k) Cbz-βAla-3-NH-phenylsulfonyl-Phe(3-AcOxAm)-Piperidinbuteryl-N-Methylamid

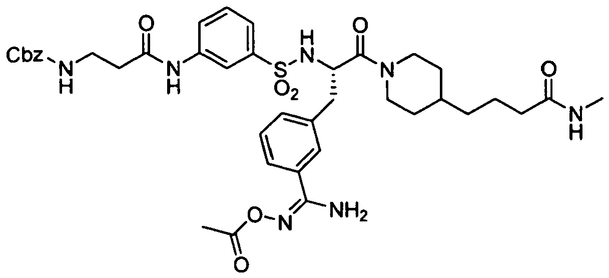

34,5 mg (0,055 mmol) Cbz-βAla-3-NH-phenylsulfonyl-Phe(3-AcOxAm)-OH und 14,9 mg (0,056 mmol) Piperidinbuteryl-N-Methylamid x HBr werden in 1,5 ml DMF gelöst und bei 0°C mit 29 mg (0,055 mmol) PyBop und 19,2 µl (0,11 mmol) DIEA versetzt. Der Ansatz wird 30 Minuten bei 0°C und 2 h bei Raumtemperatur gerührt und das Lösungsmittel i.V. entfernt. Der Rückstand wird in Essigester aufgenommen und 3 x mit 5 % KHSO₄-Lösung, 1 x mit gesättigter Kochsalzlösung, 3 x mit gesättigter NaHCO₃-Lösung und 3 x mit gesättigter Kochsalzlösung gewaschen. Die organische Phase wird über Na₂SO₄ getrocknet und im Vakuum eingeengt.
Ausbeute: 46 mg Öl, MS ber.: 791,33; gef.: 790,1 [M-H]⁻ und 814.5 [M+Na]⁺.
HPLC: 45,95 % B

### 17) H-βAla-3-NH-phenylsulfonyl-Phe(3-Am)-Piperidinbuteryl-N-Methylamid × 2 TFA

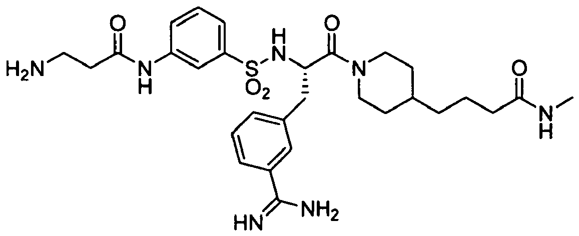

46 mg öliges Rohprodukt an Cbz-βAla-3-NH-phenylsulfonyl-Phe(3-AcOxAm)-Piperidinbuteryl-N-Methylamid werden in 50 ml Essigsäure (90 %) gelöst, mit 5 mg Katalysator (10 % Pd/C) versetzt und 64-Stunden bei Raumtemperatur mit Wasserstoff hydriert. Der Katalysator wird abfiltriert, das LM i.V. eingeengt und der Rückstand mittels präparativer HPLC gereinigt und das Produkt lyophilisiert.
Ausbeute: 24 mg lyophilisiertes Pulver, MS ber.: 599,29; gef.: 600,4 [M+H]⁺,
HPLC: 23,72 % B

In Analogie zu den beschriebenen Synthesvorschriften für die Inhibitoren 1 und 17 wurden weitere Verbindungen mit einem C-terminalem Piperidinbuttersäure-Methylamid, Methylester, Amid und freier Säurefunktion hergestellt (Tabelle 2). Der für Inhibitor 20 notwendige Ausgangsstoff 4-Piperidylbuttersäuremethylester wurde durch Umsetzung der 4-Piperidylbuttersäure mit Thionylchlorid in Methanol erhalten, das entsprechende Amid für Inhibitor 21 wurde durch Versetzen des Methylesters mit 7 N Ammoniak in Methanol (4 Tage Reaktionsdauer) hergestellt. Der Inhibitor 22 mit freier C-terminaler Säurefunktion wurde durch finale Verseifung des Inhibitors 20 mit LiOH-Lösung synthetisiert.

Die Inhibitoren 23-27a wurden in Analogie zur Synthesevorschrift von Inhibitor 17 hergestellt, wobei als Amin-Komponente analog Schritt 17k H-iNip-NH₂ (Inhibitor 23), H-Nip-NH₂ (Inhibitor 24), H-Pzd-COOEt (Inhibitor 25), H-Pzd-Cbz (Inhibitor 26 und 27) und Piperidin (Inhibitor 27a) verwendet wurden. Für die Inhibitoren 26 und 27 wurde anstelle von Cbz-ß-Ala-OH analog Schritt 17d Boc-ß-Ala-OH als Carboxylkomponente verwendet, die Abspaltung der Boc-Gruppe erfolgte im finalen Schritt durch Zugabe von 90 % TFA. Im Falle von Inhibitor 26 wurde das Amidin durch Reduktion der Acetylhydroxyamidino-Gruppe mittels Zinkstaub in AcOH erhalten. Für die Synthesen der Inhibitoren 27b-27d wurden die Lactame Pyridazinon (für 27b), Piperidinon (für 27c) und Cbz-Piperazinon (für 27d) eingesetzt, wobei die Amidierung des Aryl-Halids 3-Iodophenylsulfonyl-3-Cyanophenylalanin-4-(Methylamidobuteryl)Piperidid mittels Kupfer-katalysierter Reaktion nach bekannten Methoden erfolgte (Zusatz von ca. 1 mol% CuI, ca. 2 Äquivalente K₃PO₄, ca. 10 mol% (1R,2R)-N,N'-Dimethyl-1,2-cyclohexandiamin in DMF bei 110 °C, ca. 2 h, Klappars et al., J. Am. Chem. Soc. 2001, 123, 7727-7729).

### Beispiele 18-29:

Von den Verbindungen 18 und 19 wurden die Prodrugs mit freier Hydroxyamidino-Funktion hergestellt (Inhibitoren 28 und 29).

**Tabelle 2:**

| Beispiel/Nr. | Struktur | HPLC (% B) | MS berechnet gefunden(M+H)⁺ |
|---|---|---|---|
| 18 | 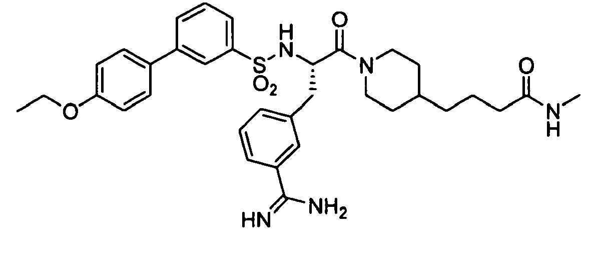 | 43,93 | 633,3 634,3 |
| 19 | 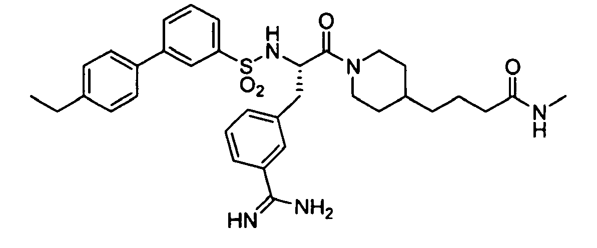 | 48,31 | 617,3 618,2 |
| 20 | 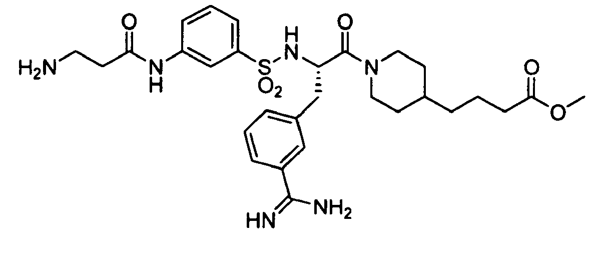 | 29,60 | 600,27 601,3 |
| 21 | 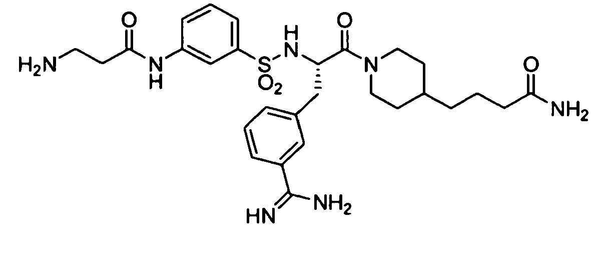 | 22,60 | 585,27 586,3 |
| 22 | 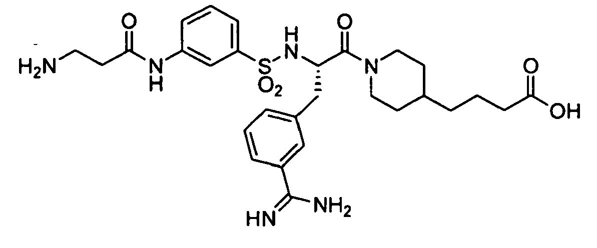 | 25,8 | 586,26 587,3 |
| 23 | 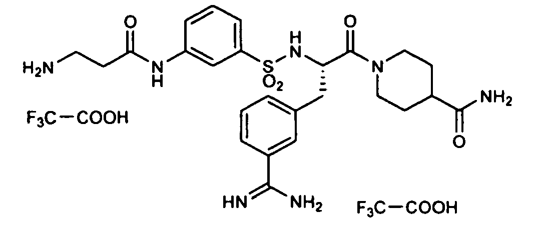 | 18.8 | 543,23 544,3 |
| 24 | 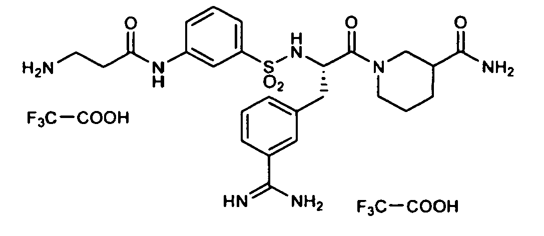 | 19,5 | 543,23 544,3 |
| 25 | 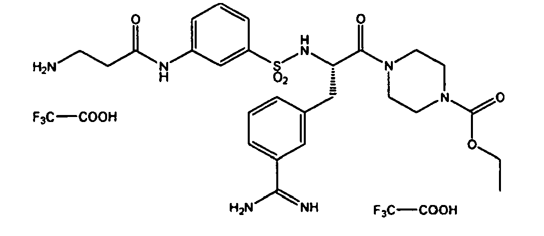 | 25,0 | 573,24 |
| 26 | 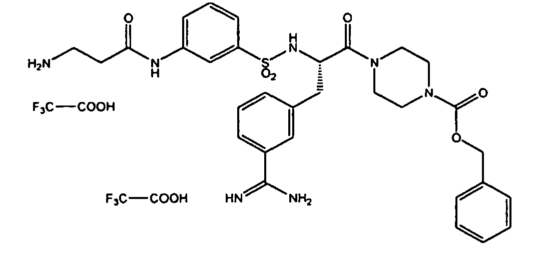 | 32,6 | 635,26 636,3 |
| 27 | 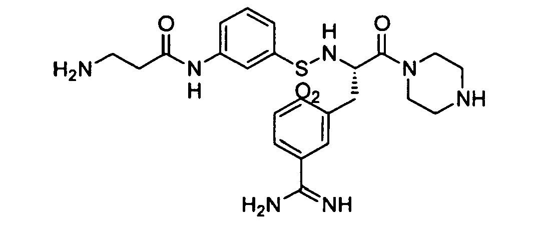 | 15,7 | 501,22 502,3 |
| 28 | 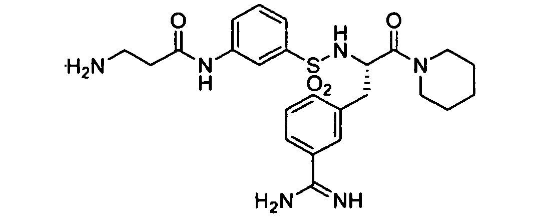 | 23,6 | 500,22 501,3 |
| 29 | 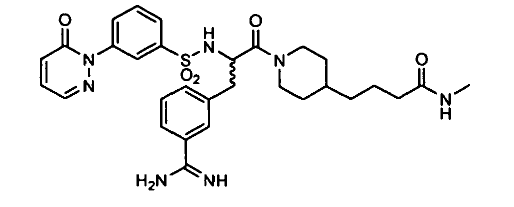 | 31.78 | 607.26 608.2 |
| 30 | 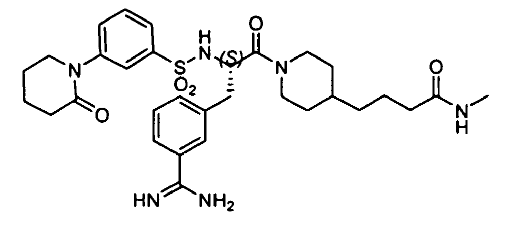 | 31,29 | 610,29 611,3 |
| 31 | 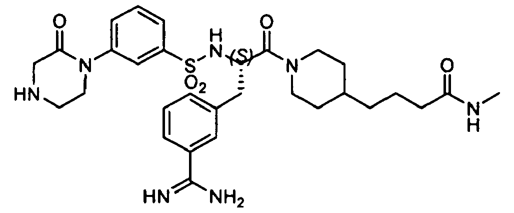 | 24.2 | 611.29 613.3 |
| Prodrugs | | | |
| 32 | 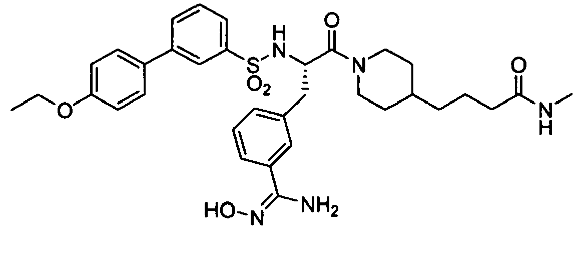 | 42,97 | 649,29 650,4 |
| 33 | 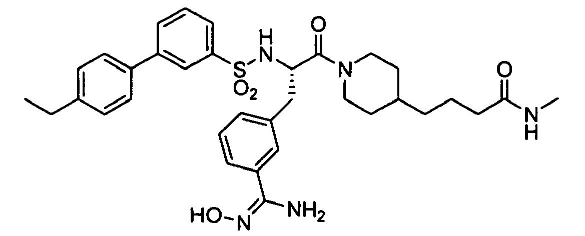 | 47,49 | 633,3 634,3 |

### Beispiel 34:

### H-βAla-3-NH-phenylsulfonyl-Phe(3-Ame)-(2-Aminoethyl)Piperidid × 3 TFA

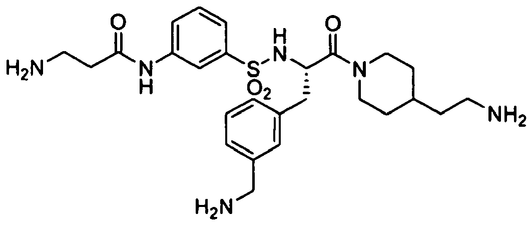

### 34a)

### 3-NH₂-Phenylsulfonyl-Phe(3-Ame)-OH x 2 HCl

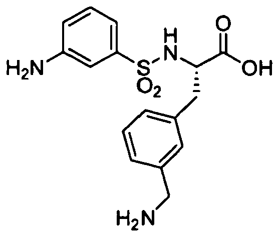

160 mg (0,425 mmol) 3-NO₂-Phenylsulfonyl-Phe(3-CN)-OH (HPLC: 45,49 % B, Steinmetzer et al., J. Med. Chem. 49, 2006, 4116) werden in 50 ml 90 % Essigsäure und 2 ml 1 N HCl gelöst und unter Normaldruck 48 h mit Wasserstoff und Pd/C als Katalysator hydriert. Das Lösungsmittel wird i.V. entfernt, der Rückstand mit Methanol gelöst und durch Zugabe von Diethylether gefällt.
Ausbeute: 133 mg Pulver, MS ber.: 349,11; gef.: 350,1 [M+H]⁺,
HPLC: 19,5% B

### 34b)

### 3-NH₂-Phenylsulfonyl-Phe(3-Boc-Ame)-OH

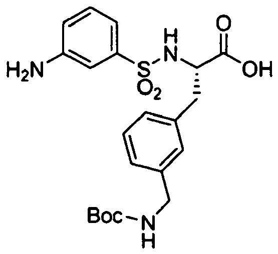

124 mg (0,294 mmol) 3-NH₂-PhenylsulfonylPhe(3-Ame)-OH x 2 HCl warden in 2 ml Dioxan und 2 ml Wasser unter Zugabe von 102 µl (0,59 mmol) DIEA gelöst. Bei 0 °C werden 70 mg (0,32 mmol) Boc-Pyrocarbonat, gelöst in 500 µl Dioxan, in mehreren Portionen zugesetzt, wobei der pH-Wert kontrolliert und auf pH 8,5-9 mit DIEA eingestellt wird. Der Ansatz wird noch weitere 15 min bei 0 °C und über Nacht bei RT gerührt. Das LM wird i.V. entfernt, der Rückstand in Essigester aufgenommen, 2 x mit NaClgesättigtem Wasser gewaschen, mit Na₂SO₄ getrocknet und das LM i.V. eingeengt.
Ausbeute: 125 mg gelbes Öl, MS ber.: 449,1; gef.: 448,1 [M-H]⁻,
HPLC: 41,6% B

### 34c)

### 3-NH₂-Phenylsulfonyl-Phe(3-Boc-Ame)-4-(2-Boc-amidoethyl)Piperidid

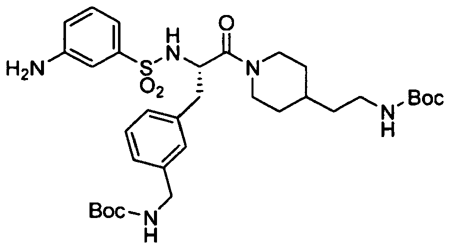

120 mg (0,267 mmol) 3-NH₂-Phenylsulfonyl-Phe(3-Boc-Ame)-OH und 64 mg (0,28 mmol) (2-Boc-amidoethyl)Piperidin werden in 4 ml DMF gelöst und mit 138 mg (0,267 mmol) PyBop und 93 µl (0,534 mmol) DIEA bei 0 °C versetzt. Der Ansatz wird 30 min bei 0 °C und über Nacht bei RT gerührt. Das LM wird i.V. entfernt, der Rückstand in Essigester gelöst und
2 x mit gesättigter NaHCO₃-Lösung und 2 x mit gesättigter Kochsalzlösung gewaschen. Die organische Phase wird über Na₂SO₄ getrocknet und im Vacuum eingeengt.
Ausbeute: 210 mg gelbbraunes Öl
HPLC: 57,38 % B

### 34) H-βAla-3-NH-phenylsulfonyl-Phe(3-Ame)-(2-Aminoethyl)Piperidid x 3 TFA

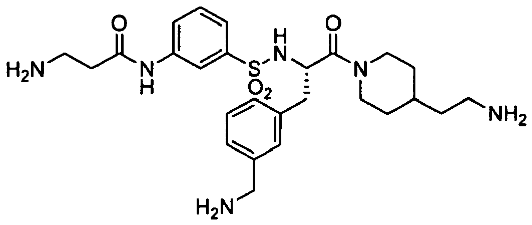

57 mg (0,3 mmol) Boc-βAla-OH werden in 2 ml DMF gelöst und bei -15°C mit 33 µl NMM und 39 µl CKIBE versetzt. Der Ansatz wird 10 min bei -15°C gerührt und danach mit 200 mg (0,303 mmol) 3-NH₂-PhenylsulfonylPhe(3-Boc-Ame)-4-(2-Bocamidoethyl)Piperidid versetzt. Der Ansatz wird über 1 h bei -15°C und über Nacht bei RT gerührt. Das LM wird i.V. entfernt, der Rückstand wird in EE aufgenommen und 3 x mit 5 % KHSO4-Lösung, 1 x mit gesättigter NaCl-Lösung, 3 x mit gesättigter NaHCO₃-Lösung und 3 x mit gesättigter NaCl-Lösung gewaschen. Das LM wird mit Na₂SO₄ getrocknet und im Vakuum entfernt. Der verbleibende Rückstand wird mit 1,5 ml TFA versetzt, 1,5 h geschüttelt und das Produkt durch preparative HPLC gereinigt, sowie lyophilisiert.
Ausbeute: 90 mg weißes lyophilisiertes Pulver, MS ber.: 530,27; gef.: 531,4 [M+H]⁺, HPLC: 18,89% B

Nach oben beschriebenen Standardverfahren wurden weitere Inhibitoren mit zentralem 3-Aminomethylphenylalanin synthetisiert (Tabelle 3)

**Tabelle 3:**

| Beispiel/Nr. | Struktur | HPLC (% B) | MS berechnet gefunden (M+H)⁺ |
|---|---|---|---|
| 35 | 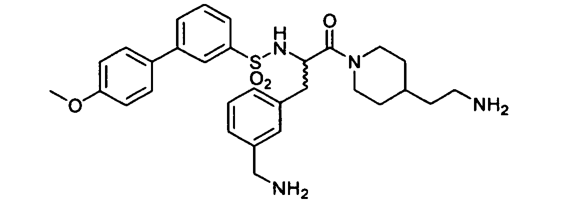 | 33,67 | 550,26 551,2 |
| 36 | 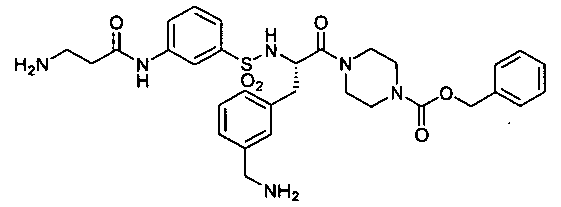 | 32,6 | 622,26 623,3 |
| 37 | 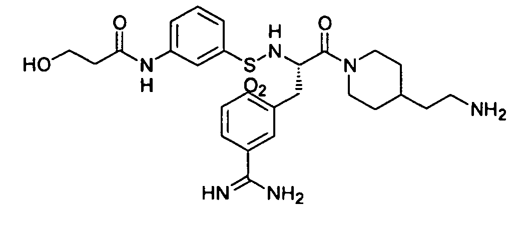 | 20,38 | 544,25 545,3 |
| 38 | 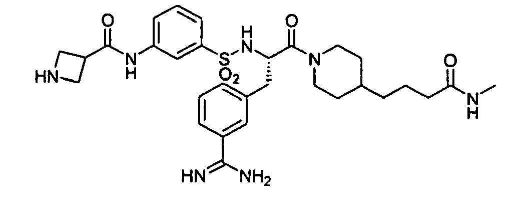 | 24,58 | 611,29 612,3 |
| 39 | 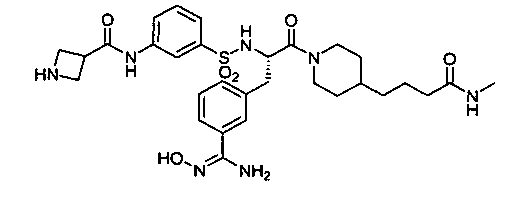 | 23,83 | 627,28 628,3 |
| 40 | 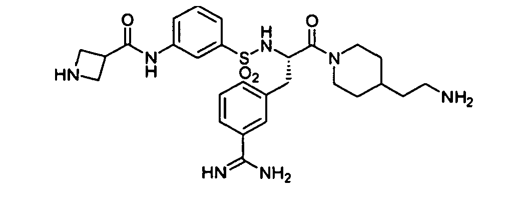 | 18,13 | 555,26 556,3 |
| 41 | 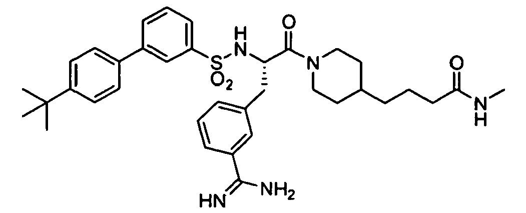 | 49,09 | 645.33 646,6 |
| 42 | 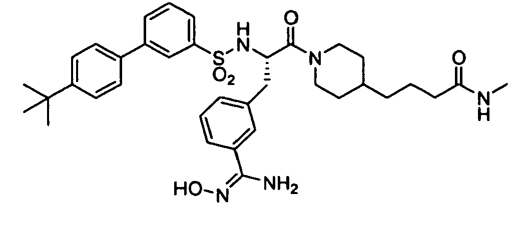 | 26,37 | 609,31 610,3 |
| 43 | 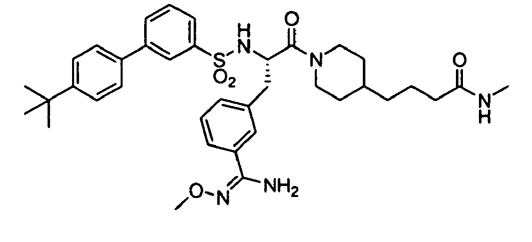 | - | 675,3 |
| 44 | 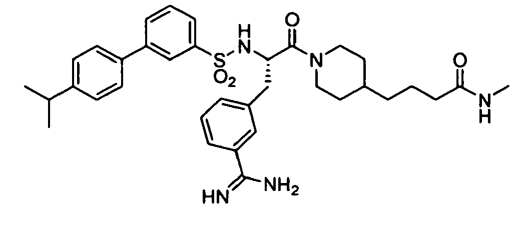 | 47,3 | 631,32 632,3 |
| 45 | 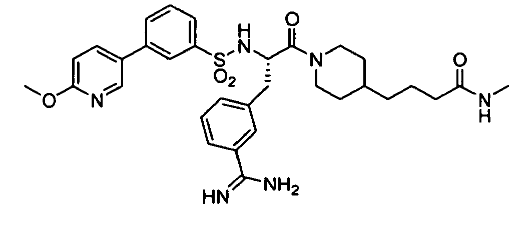 | - | 620,28 |
| 46 | 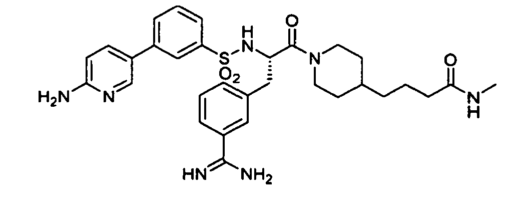 | 26,3 | 605,28 606,3 |
| 47 | 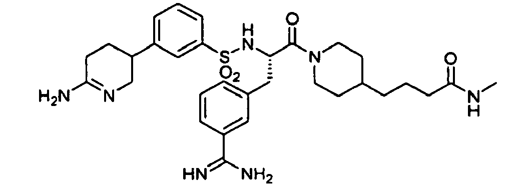 | 26,37 | 609,31 610,3 |
| 48 | 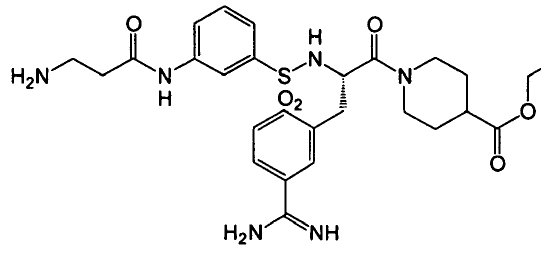 | 34,59 | 634,26 635,32 |

### PEG-gekoppelte Inhibitoren:

### Beispiel 49:

CH₃-PEG₁₀₀₀₀-CH₂-CH₂-CO-Dap-3-NH-phenylsulfonyl-Phe(3-Am)-4-(2-Aminoethyl)-Piperidid x 2 Acetat

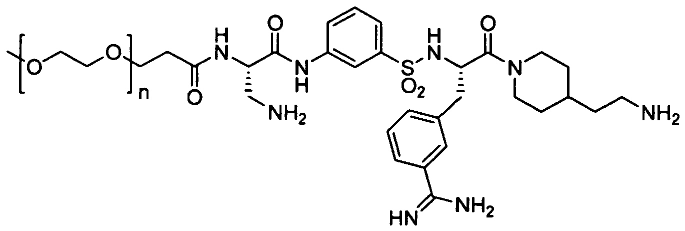

20 mg (20,3 µmol) H-Dap(Boc)-3-NH-phenylsulfonyl-Phe(3-Am)-4-(2-Boc-Amidoethyl)Piperidid x 2 TFA und 7,05 µl DIEA (40,5 mmol) wurden in 1 ml DMF und 2 ml ACN gelöst und bei Raumtemperatur mit 203 mg (ca. 20 µmol) mPEG-SPA-10kDa (Nektar Therapeutics, USA) versetzt. Der Ansatz wurde über Nacht gerührt und das Lösungsmittel i.V. entfernt. Der Rückstand wurde mit wenig Methanol angelöst, das Zwischenprodukt mit Diethylether gefällt und abgesaugt (HPLC auf Jupiter Säule: 49,85 % B). Das Zwischenprodukt wurde mit 3 ml TFA versetzt, 1h geschüttelt, das LM i.V. eingeengt, der Rückstand in wenig Methanol gelöst, mit Diethylether gefällt und das Rohprodukt abgesaugt. Das Produkt wurde mittels Ionenaustauschchromatographie an Fractogel CE und einem Ammoniumacetat-Gradienten gereinigt und 3 x aus Wasser lyophilisiert.
Ausbeute: 82 mg weißes lyophilisiertes Pulver
MS beispielsweise für n = 240: ber.: 11217.4; gef.: 11218.6 [M+H]⁺
HPLC: 44,79 % B auf Jupiter-Säule

### Beispiel 50:

### H-βAla-3-NH-phenylsulfonyl-Phe(3-Am)-4-Piperidyl-(CH₂)₃-CONH-(CH₂)₂-PEG10000-CH₃ x 2 Acetat

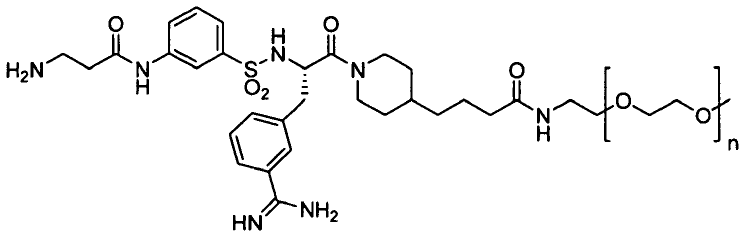

11,5 mg (13,8 µmol) Cbz-βAla-3-NH-phenylsulfonyl-Phe(3-Am)-4-Piperidinbuttsäure x TFA und 139 mg NH₂-PEG₁₀₀₀₀-CH3 (Rapp Polymer, Deutschland) werden in 3 ml DMF und 1 ml ACN gelöst und bei 0 °C mit 8 mg PyBop und 5 µl DIEA versetzt. Der Ansatz wird 15 min bei 0 °C und über Nacht bei RT gerührt. Das LM wird i.V. entfernt und der Rückstand (HPLC auf Jupiter-Säule: 47,44 % B) in 5 ml 90 % Essigsäure gelöst und über Nacht mit Wasserstoff und Pd/C als Katalysator hydriert. Der Katalysator wird abfiltriert, das LM i.V. eingeengt, in wenig Methanol gelöst und durch Zugabe von Diethylether gefällt. Das abgesaugte Rohprodukt wurde mittels Ionenaustauschchromatographie an Fractogel CE und einem Ammoniumacetat-Gradienten gereinigt und 3 x aus Wasser lyophilisiert.
Ausbeute: 22 mg weißes lyophilisiertes Pulver
HPLC: 45,77 % B auf Jupiter-Säule

Mittels Standardverfahren wurden weitere PEG-gekoppelte Inhibitoren hergestellt (Tabelle 4).

**Tabelle 4:**

| Beispiel/ Nr. | Struktur | HPLC (%B) |
|---|---|---|
| 51 | 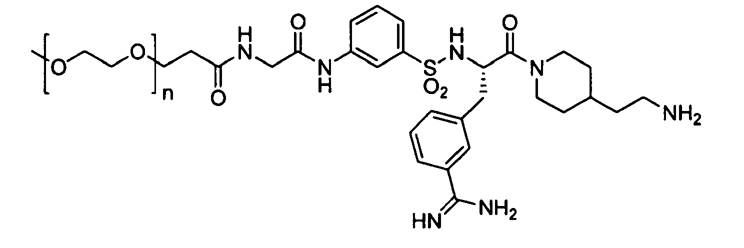 | 45,65 |
| 52 | 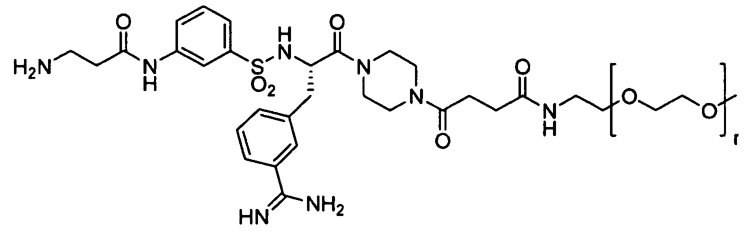 | 44,6 |

### Bestimmung der Matriptase-Hemmung

Zur Bestimmung der Hemmwirkung wurden 200 µl Tris-Puffer (0,05 M, 0,154 M NaCI, 5 % Ethanol, pH 8,0; enthält den Inhibitor), 25 µl Substrat (CH₃SO₂-D-Cha-Gly-Arg-pNA; 2 und 1mM) und 50 µl Matriptase (0,5 µg/ml) bei 25 °C inkubiert. Nach 3 min wurde die Reaktion durch Zugabe von 25 µl Essigsäure (50%) unterbrochen und die Absorption bei 405 nm mittels eines Microplate Reader (Multiscan Ascent der Firma Thermo Electron Corporation) bestimmt. Die Kᵢ-Werte wurden nach Dixon (Biochem. J. 55, 170-171, 1953) durch lineare Regression mittels eines Computerprogramms ermittelt. Die Kᵢ-Werte sind das Mittel aus mindestens zwei Bestimmungen.
Ki-Werte für die Hemmung der Matriptase in nM (n.b. = nicht bestimmt)

| Beispiel/Nr. | Ki (nM) |
|---|---|
| Inhibitor 1 | 5,4 |
| Inhibitor 2: | 100 |
| Inhibitor 3: | 6,0 |
| Inhibitor 4: | 5,4 |
| Inhibitor 5: | 2,5 |
| Inhibitor 6: | 12 |
| Inhibitor 7: | 26 |
| Inhibitor 8: | 91 |
| Inhibitor 9: | 29 |
| Inhibitor 10: | 60 |
| Inhibitor 11: | 47 |
| Inhibitor 12: | 28 |
| Inhibitor 13: | 13 |
| Inhibitor 14: | 4,2 |
| Inhibitor 15: | 0,069 |
| Inhibitor 16: | 0,74 |
| Inhibitor 17: | 6,3 |
| Inhibitor 18: | 8,1 |
| Inhibitor 19: | 24,5 |
| Inhibitor 20: | 6,1 |
| Inhibitor 21: | 11,8 |
| Inhibitor 22: | 255 |
| Inhibitor 23: | 12,1 |
| Inhibitor 24 | 30,5 |
| Inhibitor 25 | 65 |
| Inhibitor 26 | 6,1 |
| Inhibitor 27 | 36 |
| Inhibitor 28 | 32 |
| Inhibitor 29 | 98 |
| Inhibitor 30 | 31 |
| Inhibitor 31 | 24 |
| Inhibitor 32 | n.b. (Prodrug) |
| Inhibitor 33 | 24000 (Prodrug) |
| Inhibitor 34 | 56 |
| Inhibitor 35 | 145 |
| Inhibitor 36 | 44,5 |
| Inhibitor 37 | 117 |
| Inhibitor 38 | 16 |
| Inhibitor 39 | n.b. (Prodrug) |
| Inhibitor 40 | 1,7 |
| Inhibitor 41 | 87 |
| Inhibitor 42 | n.b (Prodrug) |
| Inhibitor 43 | n.b (Prodrug) |
| Inhibitor 44 | 23,5 |
| Inhibitor 45 | n.b. |
| Inhibitor 46 | 11 |
| Inhibitor 47 | 0,49 |
| Inhibitor 48 | 0,7 |
| Inhibitor 49 | 21,8 |
| Inhibitor 50 | 54 |
| Inhibitor 51 | 130 |
| Inhibitor 52 | 246 |

## Patentansprüche

1. Verbindung gemäß Formel (I) 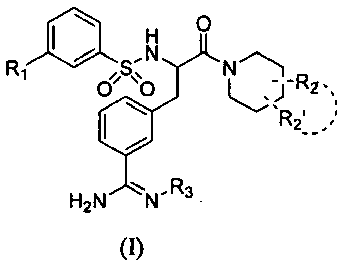 oder ein Salz dieser Verbindung, wobei
R₁ eine gegebenenfalls ein- oder mehrfach substituierte Ringstruktur ist ausgewählt aus einem Arylrest oder Heteroarylrest, die auch teilweise oder vollständig hydriert sein können; und
R₂ und R₂', sofern einzeln oder zusammen vorhanden, unabhängig voneinander sind ein verzweigter oder geradkettiger Alkylrest mit 1-6 Kohlenstoffatomen, wobei ein oder mehrere Methylengruppen durch Heteroatome ausgetauscht sein können; ein verzweigter oder geradkettiger Aminoalkylrest oder Guanidinoalkylrest mit 1-6 Kohlenstoffatomen; oder -(CH₂)ₘ⁻C(=O)-NHR₄ mit m gleich eine ganze Zahl von 0 bis 4 und R4 ein Wasserstoff oder ein -(CH₂)ₖ-CH₃ Rest mit k gleich eine ganze Zahl von 0 bis 3; oder R₂ und R₂' bilden eine Ringstruktur, die zusammen mit dem Piperidid folgendes Strukturelement bilden: 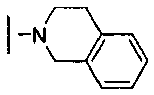 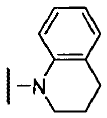 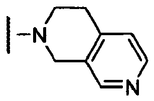 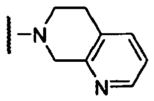 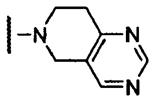 oder
R₁ ist ein verzweigter oder geradkettiger Aminoalkylrest, Hydroxyalkylrest mit 1 bis 6 Kohlenstoffatomen; oder ein 3-Azetidin-C(=O)-NH-Rest; und
R₂ und R₂', sofern einzeln oder zusammen vorhanden, unabhängig voneinander sind ein verzweigter oder geradkettiger Aminoalkylrest oder Guanidinoalkylrest mit 1-6 Kohlenstoffatomen; oder R₂ ist -(CH₂)ₘ-C(=O)-NHR₄ mit m gleich eine ganze Zahl von 0 bis 4 und R₄ ein Wasserstoff oder ein -(CH₂)ₖ-CH₃ Rest mit k gleich eine ganze Zahl von 0 bis 3;
oder
R₁ ist ein H₂N-(CH₂)ₙ-C(=O)-NH-Rest, ein HO-(CH₂)ₙ-C(=O)-NH-Rest mit n gleich eine ganze Zahl von 1 bis 4; oder ein 3-Azetidin-C(=O)-NH-Rest; und
R₂ und R₂', sofern einzeln oder zusammen vorhanden, unabhängig voneinander sind ein nicht basischer Rest; oder R₂ und R₂' bilden eine Ringstruktur, die zusammen mit dem Piperidid eines der oben genannten Strukturelemente bilden;
und
R₃ ist in allen obigen Fällen ein Wasserstoff, eine Hydroxylgruppe, eine Alkoxygruppe, eine Acetyloxygruppe oder eine Alkyloxycarbonylgruppe, wobei der Alkylrest 1-6 Kohlenstoffatome besitzt.

2. Verbindung nach Anspruch 1, bei denen R₂' nicht vorhanden ist, **dadurch gekennzeichnet, dass**
R₁ ausgewählt ist aus einem gegebenenfalls ein- oder mehrfach substituierten Phenylrest, Pyridylrest, Pyrimidinrest, Indolrest, Tetrahydropyridylrest, Piperidinonrest oder Pyridazinonrest; und
R₂ ist ein geradkettiger Aminoalkylrest mit 1-6 Kohlenstoffatomen; oder -(CH₂)ₘ-C(=O)-NHR₄ mit m gleich eine ganze Zahl von 0 bis 4 und R₄ ein Wasserstoff oder ein -(CH₂)ₖ-CH₃ Rest mit k gleich eine ganze Zahl von 0 bis 3;
oder
R₁ und R₂ sind ein geradkettiger Aminoalkylrest mit 1 bis 6 Kohlenstoffatomen; oder
R₁ ist ein H₂N-(CH₂)ₙ-C(=O)-NH-Rest mit n gleich eine ganze Zahl von 1 bis 4; und
R₂ ist ausgewählt aus folgenden Resten: -(CH₂)ₘ-C(=O)-NHR₄ mit m gleich eine ganze Zahl von 0 bis 4 und R₄ ein Wasserstoff oder ein -(CH₂)ₖ-CH₃ Rest mit k gleich eine ganze Zahl von 0 bis 3; oder -(CH₂)₀-C(=O)-OR₅ mit o eine ganze Zahl von 1 bis 6 und R₅ ein Wasserstoff oder eine geradkettige Alkylgruppe mit 1 bis 4 Kohlenstoffatomen;
und
R₃ ist in allen obigen Fällen ein Wasserstoff, eine Hydroxylgruppe, eine Alkoxygruppe, eine Acetyloxygruppe oder eine Alkyloxycarbonylgruppe, wobei der Alkylrest 1-6 Kohlenstoffatome besitzt.

3. Verbindung nach Anspruch 1 oder 2, **dadurch gekennzeichnet, dass** der Rest R₂ in meta- oder para-Position vorliegt.

4. Verbindung nach einem der Ansprüche 1-3, **dadurch gekennzeichnet, dass** die Substitution an der Ringstruktur vom Rest R₁ ausgewählt ist aus folgenden Resten: R₆-O- mit R₆ ein verzweigter oder geradkettiger Alkylrest mit 1 bis 6 Kohlenstoffatomen; (CH₃)-(CH₂)ₚ- mit p gleich eine ganze Zahl von 0 bis 6 ist; ein Halogen; und/oder eine Aminogruppe.

5. Verbindung nach einem der Ansprüche 1-4, **dadurch gekennzeichnet, dass** die Substitution am Phenylrest ausgewählt ist aus folgenden Resten: R₆-O- mit R₆ ein verzweigter oder geradkettiger Alkylrest mit 1 bis 6 Kohlenstoffatomen; (CH₃)-(CH₂)ₚ- mit p gleich eine ganze Zahl von 0 bis 6; und/oder ein Halogen.

6. Verbindung nach einem der Ansprüche 1-4, **dadurch gekennzeichnet, dass** die Substitution am Pyridylrest, am Pyrimidinrest oder am Tetrahydropyridylrest ausgewählt ist aus folgenden Resten: (CH₃)-(CH₂)ₚ- mit p gleich eine ganze Zahl von 0 bis 6; und/oder eine Aminogruppe.

7. Verbindung gemäß Formel (II) 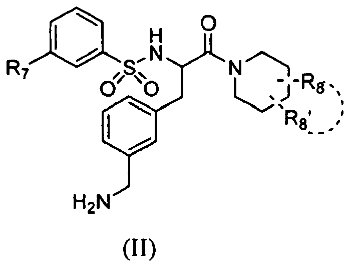 oder ein Salz dieser Verbindung, wobei
R₇ eine gegebenenfalls ein oder mehrfach substituierte Ringstruktur ist ausgewählt aus einem Arylrest oder Heteroarylrest, die gegebenenfalls teilweise oder vollständig aufhydriert ist; ein verzweigter oder unverzweigter Alkylrest mit 1-8 Kohlenstoffatomen, wobei eine oder mehrere Methylengruppen durch Heteroatome ausgetauscht sein können; ein H₂N-(CH₂),-C(=O)-NH-Rest, ein HO-(CH₂)ᵣ-C(=O)-NH-Rest, ein NH₂-(CH₂)_{q}-Rest, oder ein HO-(CH₂)_{q}-Rest, mit r gleich eine ganze Zahl von 1 bis 4 und q unabhängig voneinander gleich eine ganze Zahl von 1 bis 5 ist; oder ein 3-Azetidin-C(=O)-NH-Rest; und
R₈ und R₈', sofern einzeln oder zusammen vorhanden, unabhängig voneinander sind ein verzweigter oder geradkettiger Alkylrest, mit 1-8 Kohlenstoffatomen, wobei eine oder mehrere Methylengruppen durch Heteroatome ausgetauscht sein können; ein Aminoalkylrest oder Guanidinoalkylrest mit jeweils 1-6 Kohlenstoffatomen; oder ein -(CH₂)ₘ-C(=O)-NHR4 mit m gleich eine ganze Zahl von 0 bis 4, und R4 ein Wasserstoff oder ein -(CH₂)ₖ-CH₃ Rest mit k gleich eine ganze Zahl von 0 bis 3; oder R₈ und R₈' bilden eine Ringstruktur, die zusammen mit dem Piperidid ein Strukturelement bilden.

8. Verbindung nach Anspruch 7, bei denen R₈' nicht vorhanden ist, **dadurch gekennzeichnet, dass**
R₇ eine gegebenenfalls ein oder mehrfach substituierte Ringstruktur ist ausgewählt aus einem Phenylrest, Pyridylrest, Pyrimidinrest, Indolrest, Tetrahydropyridyl-rest, Piperidinonrest oder Pyridazinonrest; ein H₂N-(CH₂)ᵣ-C(=O)-NH-Rest, mit r gleich eine ganze Zahl von 1 bis 4; und
R₈ ist ein Aminoalkylrest mit jeweils 1-6 Kohlenstoffatomen.

9. Verbindung gemäß Formel (III) 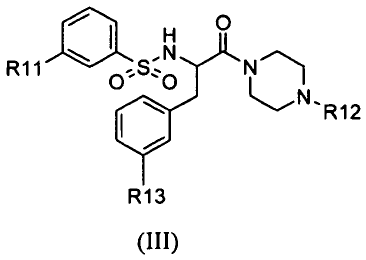 oder ein Salz dieser Verbindung, wobei
R11 ein NH₂-(CH₂)ᵥ-C(=O)-NH-Rest ist mit v gleich eine ganze Zahl von 1 bis 4; ein 3-Azetidin-C(=O)-NH-Rest, eine gegebenenfalls ein- oder mehrfach substituierte Ringstruktur ist ausgewählt aus einem Arylrest oder Heteroarylrest, die auch teilweise oder vollständig hydriert sein können;
R12 ist ein Wasserstoff; ein verzweigter oder geradkettiger Aminoalkylrest mit 1 bis 6 Kohlenstoffatomen; eine -C(=O)-O-R14 Gruppe, -C(=O)-R14 Gruppe oder - C(=O)-NH-R14 Gruppe mit jeweils R14 unabhängig voneinander gleich ein Alkylrest mit 1 bis 6 Kohlenstoffatomen; oder ein Aralkylrest oder ein Heteroaralkylrest mit jeweils 4-12 Kohlenstoffatomen in deren Aryl- oder Heteroarylsegment, wobei das Heteroarylsegment des Heteroaralkylrestes 1 bis 2 Heteroatome enthalten kann, und mit jeweils 1 bis 6 Kohlenstoffatomen im Alkylrest; und
R13 ist ein Aminomethylrest oder ein substituierter oder unsubstituierter Amidinorest der Formel -C(=NR15)NH₂ mit R15 gleich Wasserstoff, eine Hydroxylgruppe, eine Alkoxygruppe, eine Acetyloxygruppe oder eine Alkyloxycarbonylgruppe, wobei der Alkylrest 1-6 Kohlenstoffatome besitzt.

10. Verbindung nach Anspruch 9, **dadurch gekennzeichnet, dass**
R11 ein NH₂-(CH₂)ᵥ-C(=O)-NH-Rest ist mit v gleich eine ganze Zahl von 1 bis 4;
R12 ist ein Wasserstoff; ein geradkettiger Aminoalkylrest mit 1 bis 6 Kohlenstoffatomen; oder eine -C(=O)-O-R14 Gruppe mit jeweils R14 unabhängig voneinander gleich ein geradkettiger Alkylrest mit 1 bis 6 Kohlenstoffatomen; oder ein Benzylrest; und
R13 ist ein Aminomethylrest oder ein substituierter oder unsubstituierter Amidinorest der Formel -C(=NR15)NH₂ mit R15 gleich Wasserstoff, eine Hydroxylgruppe, eine Alkoxygruppe, eine Acetylhydroxylgruppe oder eine Alkyloxycarbonylgruppe, wobei der Alkylrest 1-6 Kohlenstoffatome besitzt.

11. Verbindung nach mindestens einem der Ansprüche 1-10, **dadurch gekennzeichnet, dass** an die Verbindung gegebenenfalls mit Hilfe eines Linkers eine Polyethylenglykol-Kette kovalent gekoppelt ist.

12. Verbindung nach mindestens einem der Ansprüche 1-11, **dadurch gekennzeichnet, dass** die als zentrales Strukturmotif in den Formeln (I), (II) und (III) vorhandene Aminosäure in der L-Konfiguration vorliegt.

13. Verbindung nach Anspruch 1, wobei die Verbindung ausgewählt ist aus einer der folgenden Strukturen:
3-(3,4-Dimethoxyphenyl)phenylsulfonyl-L-Phenylalanin(3-Amidino)-4-(2-Aminoethyl)Piperidid,
3-(Phenyl)phenylsulfonyl-L-Phenylalanin(3-Amidino)-4-(2-Aminoethyl)Piperidid,
3-(4-Methoxyphenyl)phenylsulfonyl-L-Phenylalanin(3-Amidino)-4-(2-Aminoethyl)Piperidid,
3-(4-Ethoxyphenyl)phenylsulfonyl-L-Phenylalanin(3-Amidino)-4-(2-Aminoethyl)Piperidid,
3-(4-Ethylphenyl)phenylsulfonyl-L-Phenylalanin(3-Amidino)-4-(2-Aminoethyl)Piperidid,
3-(4-Isopropoxyphenyl)phenylsulfonyl-L-Phenylalanin(3-Amidino)-4-(2-Aminoethyl)Piperidid,
3-(4-Chlorphenyl)phenylsulfonyl-L-Phenylalanin(3-Amidino)-4-(2-Aminoethyl)Piperidid,
3-(3-Chlorphenyl)phenylsulfonyl-L-Phenylalanin(3-Amidino)-4-(2-Aminoethyl)Piperidid,
3-(2-Chlorphenyl)phenylsulfonyl-L-Phenylalanin(3-Amidino)-4-(2-Aminoethyl)Piperidid,
3-(4-Pyridyl)phenylsulfonyl-L-Phenylalanin(3-Amidino)-4-(2-Aminoethyl)Piperidid,
3-(3-Pyridyl)phenylsulfonyl-L-Phenylalanin(3-Amidino)-4-(2-Aminoethyl)Piperidid,
3-(2-Methyl-4-pyrimidinyl)phenylsulfonyl-L-Phenylalanin(3-Amidino)-4-(2-Aminoethyl)Piperidid,
3-(5-Indolyl)phenylsulfonyl-L-Phenylalanin(3-Amidino)-4-(2-Aminoethyl)Piperidid,
3-(4-Amino-3-pyridyl)phenylsulfonyl-L-Phenylalanin(3-Amidino)-4-(2-Aminoethyl)Piperidid,
3-(4-Amino-3-Tetrahydropyridyl)phenylsulfonyl-L-Phenylalanin(3-Amidino)-4-(2-Aminoethyl)Piperidid,
3-(1-Aminobutyl)-Phenylsulfonyl-L-Phenylalanin(3-Amidino)-4-(2-Aminoethyl)Piperidid,
3-(H-β-Alanyl-NH)phenylsulfonyl-L-Phenylalanin(3-Amidino)-4-(Methylamidobuteryl)Piperidid,
3-(4-Ethoxyphenyl)phenylsulfonyl-L-Phenylalanin(3-Amidino)- 4-(Methylamidobuteryl)Piperidid,
3-(4-Ethylphenyl)phenylsulfonyl-L-Phenylalanin(3-Amidino)- 4-(Methylamidobuteryl)Piperidid,
3-(H-β-Alanyl-NH)phenylsulfonyl-L-Phenylalanin(3-Amidino)-4-Piperidinbuteryl-O-Methylat,
3-(H-β-Alanyl-NH)phenylsulfonyl-L-Phenylalanin(3-Amidino)-4-(Amidobuteryl)Piperidid,
3-(H-β-Alanyl-NH)phenylsulfonyl-L-Phenylalanin(3-Amidino)- 4-(Buteryl)Piperidid,
3-(H-β-Alanyl-NH)phenylsulfonyl-L-Phenylalanin(3-Amidino)-Isonipecotylamid,
3-(H-β-Alanyl-NH)phenylsulfonyl-L-Phenylalanin(3-Amidino)-Nipecotylamid,
3-(H-β-Alanyl-NH)phenylsulfonyl-L-Phenylalanin(3-Amidino)-Piperidid,
3-(Pyridazinon)phenylsulfonyl-L-Phenylalanin(3-Amidino)-4-(Methylamidobuteryl) Piperidid,
3-(Piperidinon)phenylsulfonyl-L-Phenylalanin(3-Amidino)-4-(Methylamidobuteryl) Piperidid,
3-(Piperazinon)phenylsulfonyl-L-Phenylalanin(3-Amidino)-4-(Methylamidobuteryl) Piperidid,
3-(4-Ethoxyphenyl)phenylsulfonyl-L-Phenylalanin(3-Hydroxyamidino)-Piperidinbuteryl-N-Methylamid,
3-(4-Ethylphenyl)phenylsulfonyl-L-Phenylalanin(3-Hydroxyamidino)-Piperidinbuteryl-N-Methylamid,
3-(1-Hydroxybutyl)-Phenylsulfonyl-L-Phenylalanin(3-Amidino)-4-(2-Aminoethyl) Piperidid,
3-(3-Azetidin-CONH)-Phenylsulfonyl-L-Phenylalanin(3-Amidino)-4-(Methylamidobuteryl)Piperidid,
3-(3-Azetidin-CONH)-Phenylsulfonyl-L-Phenylalanin(3-Hydroxyamidino)-4-(Methylamidobuteryl)Piperidid,
3-(3-Azetidin-CONH)-Phenylsulfonyl-L-Phenylalanin(3-Amidino)-4-(2-Aminoethyl)Piperidid,
3-(4-tert.Butylphenyl)phenylsulfonyl-L-Phenylalanin(3-Amidino)-4-(Methylamidobuteryl)Piperidid,
3-(4-tert.Butylphenyl)phenylsulfonyl-L-Phenylalanin(3-Hydroxyamidino)-4-(Methylamidobuteryl)Piperidid,
3-(4-tert.Butylphenyl)phenylsulfonyl-L-Phenylalanin(3-Methoxyamidino)-4-(Methylamidobuteryl)Piperidid,
3-(4-Isopropylphenyl)phenylsulfonyl-L-Phenylalanin(3-Amidino)-4-(Methylamidobuteryl)Piperidid,
3-(4-Methoxy-3-pyridyl)phenylsulfonyl-L-Phenylalanin(3-Amidino)-4-(Methylamidobuteryl)Piperidid,
3-(4-Amino-3-Pyridyl)phenylsulfonyl-L-Phenylalanin(3-Amidino)-4-(Methylamidobuteryl)Piperidid,
3-(4-Amino-3-Tetrahydropyridyl)phenylsulfonyl-L-Phenylalanin(3-Amidino)-4-(Methylamidobuteryl)Piperidid,
3-(Na-(CH₃-PEG₁₀₀₀₀-CH₂-CH₂-CO)-α,β-Diaminopropionyl-NH)phenylsulfonyl-L-Phenylalanin(3-Amidino)-4-(2-Aminoethyl)-Piperidid,
3-(H-β-Alanyl-NH)phenylsulfonyl-L-Phenylalanin(3-Amidino)-4-(CH3-PEG 10000-CH₂-CH₂-amidobuteryl)Piperidid,
3-(CH₃-PEG₁₀₀₀₀-CH₂-CH₂-CO)-β-Ala-NH)phenylsulfonyl-L-Phenylalanin(3-Amidino)-4-(2-Aminoethyl)-Piperidid,
3-(H-β-Alanyl-NH)phenylsulfonyl-L-Phenylalanin(3-Amidino)-Nipecotyl-benzylester.

14. Verbindung nach Anspruch 7, wobei die Verbindung ausgewählt ist aus einer der folgenden Strukturen:
3-(H-βAlanin-NH)phenylsulfonyl-L-Phenylalanin(3-Aminomethyl)-4-(2-Aminoethyl) Piperidid,
3-(4-Methoxyphenyl)phenylsulfonyl-L-Phenylalanin(3-Aminomethyl)-4-(2-Aminoethyl)Piperidid.

15. Verbindung nach Anspruch 9, wobei die Verbindung ausgewählt ist aus einer der folgenden Strukturen:
3-(H-β-Alanyl-NH)phenylsulfonyl-L-Phenylalanin(3-Amidino)-4-(Ethyloxycarbonyl) Piperazid,
3-(H-β-Alanyl-NH)phenylsulfonyl-L-Phenylalanin(3-Amidino)-4-Benzyloxycarbonyl) Piperazid,
3-(H-β-Alanyl-NH)phenylsulfonyl-L-Phenylalanin(3-Amidino)-Piperazid,
3-(H-β-Alanyl-NH)phenylsulfonyl-L-Phenylalanin(3-Aminomethyl)-4-(Benzyloxycarbonyl)Piperazid,
3-(H-β-Alanyl-NH)phenylsulfonyl-L-Phenylalanin(3-Amidino)-4-(CH3-PEG₁₀₀₀₀-CH₂-CH₂-NH-Suc)Piperazid

16. Arzneimittel enthaltend mindestens eine der Verbindungen gemäß einem der Ansprüche 1-15.

17. Arzneimittel nach Anspruch 16 zur Reduktion der Bildung von Tumormetastasen.

## Claims

1. A compound according to formula (I) 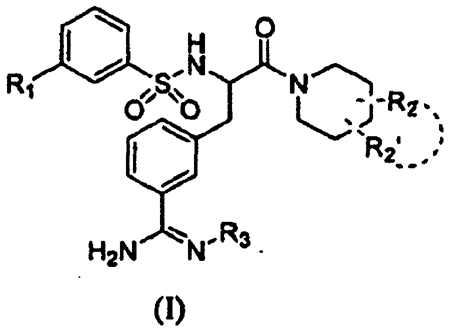 or a salt of said compound, wherein
R₁ is an optionally monosubstituted or polysubstituted ring structure selected from an aryl residue or a heteroaryl residue, which can also be partly or entirely hydrogenated; and
R₂ and R₂', as far as present soly or together, independently from another are a branched or linear chain alkyl residue with 1-6 carbon atoms, wherein one or more methylene groups can be replaced by heteroatoms; a branched or linear chain aminoalkyl residue or guanidinoalkyl residue with 1-6 carbon atoms; or -(CH₂)ₘ-C(=O)-NHR₄ wherein m equals to an integral number of from 0 to 4 and R₄ is a hydrogen or a -(CH₂)ₖ-CH₃ residue wherein k equals to an integral number of from 0 to 3; or R₂ and R₂' form a ring structure forming the following structure element together with the piperidide: 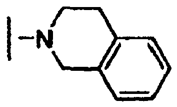 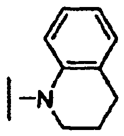 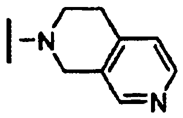 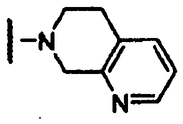 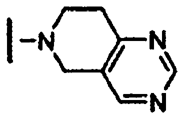 or
R₁ is a branched or linear chain aminoalkyl residue, hydroxyalkyl residue with 1 to 6 carbon atoms; or a 3-azetidine-C(=O)-NH residue; and
R₂ and R₂', as far as present soly or together, independently from another are a branched or linear chain aminoalkyl residue with 1-6 carbon atoms; or R₂ is - (CH₂)ₘ-C(=O)-NHR₄ wherein m equals to an integral number of from 0 to 4 and R₄ is a hydrogen or a -(CH₂)ₖ-CH₃ residue wherein k equals to an integral number of from 0 to 3;
or
R₁ is a H₂N-(CH₂)ₙ-C(=O)-NH residue, an HO-(CH₂)ₙ-C(=O)-NH residue wherein n equals to an integral number of from 1 to 4; or a 3-azetidine -C(=O)-NH residue; and
R₂ and R₂', as far as present soly or together, independently from another are a non-basic residue; or R₂ and R₂' form a ring structure forming together with the piperidide one of the above structure elements;
and
R₃ is, in all above cases, a hydrogen, a hydroxyl group, an alkoxy group, an acetyloxy group, or an alkyloxycarbonyl group, wherein the alkyl residue has 1-6 carbon atoms.

2. The compound of claim 1, wherein R₂' is not present, **characterized in that** R₁ is selected from an optionally monosubstituted or polysubstituted phenyl residue, pyridyl residue, pyrimidine residue, indole residue, tetrahydropyridyl residue, piperidinone residue or pyridazinone residue; and
R₂ is a linear chain aminoalkyl residue with 1-6 carbon atoms; or -(CH₂)ₘ-C(=O)-NHR₄ wherein m equals to an integral number of from 0 to 4 and R₄ is hydrogen or a -(CH₂)ₖ-CH₃ residue wherein k equals to an integral number of from 0 to 3;
or
R₁ and R₂ are a linear chain aminoalkyl residue with 1 to 6 carbon atoms;
or
R₁ is a H₂N-(CH₂-C(=O)-NH residue wherein n equals to an integral number of from 1 to 4; and
R₂ is selected from the following residues:
-(CH₂)ₘ-C(=O)-NHR₄ wherein m equals to an integral number of from 0 to 4 and R₄ is hydrogen or a -(CH₂)ₖ-CH₃ residue wherein k equals to an integral number of from 0 to 3; or -(CH₂)₀-C(=O)-OR₅ wherein o equals to an integral number of from 1 to 6 and R₅ is a hydrogen or a linear chain alkyl group with 1 to 4 carbon atoms;
and
R₃ is in all above cases a hydrogen, a hydroxyl group, an alkoxy group, an acetyloxy group or an alkyloxycarbonyl group, wherein the alkyl residue has 1-6 carbon atoms.

3. The compound of claim 1 or 2, **characterized in that** the residue R₂ is present in the meta or para position.

4. The compound of any one of claims 1-3, **characterized in that** the substitution of the ring structure of residue R₁ is selected from the following residues;
R₆-O- wherein R₆ is a branched or linear chain alkyl residue with 1 to 6 carbon atoms; (CH₃)-(CH₂)p- wherein p equals to an integral number of from 0 to 6; a halogen; and/or an amino group.

5. The compound of any one of claims 1-4, **characterized in that** the substitution at the phenyl residue is selected from the following residues: R₆-O- wherein R₆ is a branched or linear chain alkyl residue with 1 to 6 carbon atoms; (CH₃)-(CH₂)ₚ-wherein p equals to an integral number of from 0 to 6; and/or a halogen.

6. The compound of any one of claims 1-4, **characterized in that** the substitution at the pyridyl residue, at the pyrimidine residue or at the tetrahydropyridyl residue is selected from the following residues: (CH₃)-(CH₂)ₚ- wherein p equals to an integral number of from 0 to 6; and/or an amino group.

7. The compound according to formula (II) 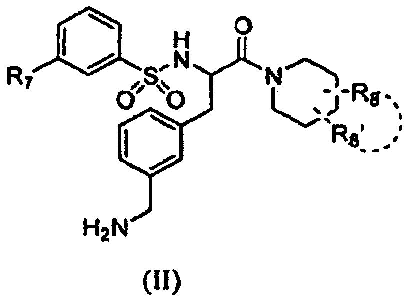 or a salt of said compound, wherein
R₇ is an optionally monosubstituted or polysubstituted ring structure selected from an aryl residue or a heteroaryl residue, which is optionally partly or entirely hydrogenated; a branched or unbranched alkyl residue with 1-8 carbon atoms, wherein one or more methylene groups can be replaced by heteroatoms; a H₂N-(CH₂)ᵣ-C(=O)-NH residue, an HO-(CH₂)ᵣ-C(=O)-NH residue, an NH₂-(CH₂)_{q} residue, or an HO-(CH₂)_{q} residue, wherein r equals to an integral number of from 1 to 4 and q is, independent from another, an integral number of from 1 to 5; or a 3-azetidine-C(=O)-NH residue; and
R₈ and R₈', as far as present soly or together, independently from another are a branched or linear chain alkyl residue with 1-8 carbon atoms, wherein one or more methylene groups can be replaced by heteroatoms; an aminoalkyl residue or guanidinoalkyl residue with 1-6 carbon atoms in each case; or a -(CH₂)ₘ-C(=O)-NHR₄ wherein m equals to an integral number of from 0 to 4 and R₄ is a hydrogen or a -(CH₂)ₖ-CH₃ residue wherein k equals to an integral number of from 0 to 3; or R₈ and R₈' form a ring structure forming a structure element together with the piperidide.

8. The compound of claim 7, wherein R₈' is not present, **characterized in that**
R₇ is an optionally monosubstituted or polysubstituted ring structure selected from a phenyl residue, a pyridyl residue, pyrimidine residue, indole residue, tetrahydropyridyl residue, piperidinone residue or pyridazinone residue; a H₂N-(CH₂)ᵣ-C(=O)-NH residue, wherein r equals to an integral number of from 1 to 4; and
R₈ is an aminoalkyl residue with 1-6 carbon atoms in each case.

9. Compound according to formula (III) 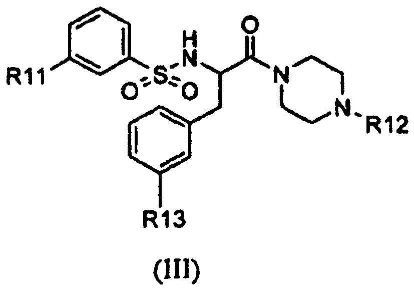 or a salt of said compound, wherein
R11 is an NH₂-(CH₂)ᵥ-C(=O)-NH residue wherein v equals to an integral number of from 1 to 4; a 3-azetidine-(C=O)-NH residue, as an optionally monosubstituted or polysubstituted ring structure selected from an aryl residue or heteroaryl residue that can also be party or entirely hydrogenated;
R12 is a hydrogen; a branched or linear chain aminoalkyl residue with 1 to 6 carbon atoms; a -C(=O)-O-R14 group, -C(=O)-R14 group or -C(=O)-NH-R14 group wherein R14 can be, independently from another, an alkyl residue with 1 to 6 carbon atoms; or an aralkyl residue or a heteroaralkyl residue of 4-12 carbon atoms in each case in their aryl or heteroaryl segment, wherein the heteroaryl segment of the heteroaralkyl residue can contain 1 to 2 heteroatoms, and with 1 to 6 carbon atoms in the alkyl residue in each case; and
R13 is an aminomethyl residue or a substituted or unsubstituted amidino residue of the formula -C(=NR15)NH₂ wherein R15 is hydrogen, a hydroxyl group, an alkoxy group, an acetyloxy group or an alkyloxycarbonyl group, wherein the alkyl residue has 1-6 carbon atoms.

10. The compound of claim 9, **characterized in that**
R11 is an NH₂-(CH₂)ᵥ-C(=O)-NH residue wherein v equals to an integral number of from 1 to 4;
R12 is a hydrogen; a linear chain aminoalkyl residue with 1 to 6 carbon atoms; or a -C(=O)-O-R14 group wherein R14 independently from another equals to a linear chain alkyl residue with 1 to 6 carbon atoms; or a benzyl residue; and
R13 is an aminomethyl residue or a substituted or unsubstituted amidino residue of the formula -C(=NR15)NH₂ wherein R15 equals to hydrogen, a hydroxyl group, an alkoxy group, an acetyl hydroxyl group or an alkyloxycarbonyl group, wherein the alkyl residue has 1-6 carbon atoms.

11. The compound of at least one of claims 1-10, **characterized in that** a polyethylene glycol chain is covalently coupled to the compound, optionally assisted by a linker.

12. The compound of at least one of claims 1-11, **characterized in that** the amino acid present as a central structure motif in the formulae (I), (II) and (III) is present in the L-configuration.

13. The compound of claim 1, wherein the compound is selected from one of the following structures:
3-(3,4-dimethoxyphenyl)phenylsulfonyl-L-phenylalanine(3-amidino)4-(2-aminoethyl)piperidide,
3-(phenyl)phenylsulfonyl-L-phenylalanine(3-amidino)-4-(2-aminoethyl)piperidide,
3-(4-methoxyphenyl)phenylsulfonyl-L-phenylalanine(3-amidino)-4-(2-aminoethyl)piperidide,
3-(4-ethoxyphenyl)phenylsulfonyl-L-phenylalanine(3-amidino)-4-(2-Aminoethyl)piperidide,
3-(4-ethylphenyl)phenylsulfonyl-L-phenylalanine(3-amidino)-4-(2-aminoethyl)piperidide,
3-(4-isopropoxyphenyl)phenylsulfonyl-L-phenylalanine(3-amidino)-4-(2-aminoethyl)piperidide,
3-(4-chlorophenyl)phenylsulfonyl-L-phenylalanine(3-amidino)-4-(2-aminoethyl)piperidide,
3-(3-chlorophenyl)phenylsulfonyl-L-phenylalanine(3-amidino)-4-(2-aminoethyl)piperidide,
3-(2-chlorophenyl)phenylsulfonyl-L-phenylalanine(3-amidino)-4-(2-aminoethyl)piperidide,
3-(4-pyridyl)phenylsulfonyl-L-phenylalanine(3-amidino)-4-(2-aminoethyl)piperidide, 3-(3-pyridyl)phenylsulfonyl-L-phenylalanine(3-amidino)-4-(2-aminoethyl)piperidide,
3-(2-methyl-4-pyrimidinyl)phenylsulfonyl-L-phenylalanine(3-amidino)-4-(2-aminoethyl)piperidide,
3-(5-indolyl)phenylsulfonyl-L-phenylalanine(3-amidino)-4-(2-aminoethyl)piperidide,
3-(4-amino-3-pyridyl)phenylsulfonyl-L-phenylalanine(3-amidino)-4-(2-aminoethyl)piperidide,
3-(4-amino-3-tetrahydropyridyl)phenylsulfonyl-L-phenylalanine(3-amidino)-4-(2-aminoethyl)piperidide,
3-(1-aminobutyl)-phenylsulfonyl-L-phenylalanine(3-amidino)-4-(2-aminoethyl)piperidide,
3-(H-ß-alanyl-NH)phenylsulfonyl-L-phenylalanine(3-amidino)-4-(methylamidobuteryl)piperidide,
3-(4-ethoxyphenyl)phenylsulfonyl-L-phenylalanine(3-amidino)-4-(methylamidobuteryl)piperidide,
3-(4-ethylphenyl)phenylsulfonyl-L-phenylalanine(3-amidino)-4-(methylamidobuteryl)piperidide,
3-(H-ß-alanyl-NH)phenylsulfonyl-L-phenylaianine(3-amidino)-4-piperidinebuteryl-O-methylate,
3-(H-ß-alanyl-NH)phenylsulfonyl-L-phenylalanine(3-amidino)-4-(amidobuteryl)piperidide,
3-(H-ß-alanyl-NH)phenylsulfonyl-L-phenylalanine(3-amidino)-4-(buteryl)piperidide,
3-(H-ß-alanyl-NH)phenylsulfonyl-L-phenylalanine(3-amidino)-isonipecotylamide,
3-(H-13-alanyl-NH)phenylsulfonyl-L-phenylalanine(3-amidmo)-nipecotylamide,
3-(H-ß-alanyl-NH)phenylsulfonyl-L-phenylalanine(3-amidino)-piperidide,
3-(pyridazinone)phenylsulfonyl-L-phenylalanine(3-amidino)-4-(methylamidobuteryl)piperidide,
3-(piperidinone)phenylsulfonyl-L-phenylalanine(3-amidino)-4-(methylamidobuteryl)piperidide,
3-(piperazinone)phenylsulfonyl-L-phenylalanine(3-amidino)-4-(methylamidobuteryl)piperidide,
3-(4-ethoxyphenyl)phenylsulfonyl-L-phenylalanine(3-hydroxyamidino)-piperidine buteryl-N-methylamide,
3-(4-ethylphenyl)phenylsulfonyl-L-phenylalanine(3-hydroxyamidino)-piperidine buteryl-N-methylamide,
3-(1-hydroxybutyl)-phenylsulfonyl-L-phenylalanine(3-amidino)-4-(2-aminoethyl) piperidide,
3-(3-azetidine-CONH)-phenylsulfonyl-L-phenylalanine(3-amidino)-4-(methylamidobuteryl)piperidide,
3-(3-azetidine-CONH)-phenylsulfonyl-L-phenylalanine(3-hydroxyamidino)-4-(Methylamidobuteryl)piperidide,
3-(3-azetidine-CONH)-phenylsulfonyl-L-phenylalanine(3-amidino)-4-(2-aminoethyl)piperidide,
3-(4-tert.-butylphenyl)phenylsulfonyl-L-phenylalanine(3-amidino)-4-(methylamidobuteryl)piperidide,
3-(4-tert.-butylphenyl)phenylsulfonyl-L-phenylalanine(3-hydroxyamidino)-4-(methylamidobuteryl)piperidide,
3-(4-tert.-butylphenyl)phenylsulfonyl-L-phenylalanine(3-methoxyamidino)-4-(methylamidobuteryl)piperidide,
3-(4-isopropylphenyl)phenylsulfonyl-L-phenylalanine(3-amidino)-4-(methylamidobuteryl)piperidide,
3-(4-methoxy-3-pyridyl)phenylsulfonyl-L-phenylalanine(3-amidino)-4-(methylamidobuteryl)piperidide,
3-(4-amino-3-pyridyl)phenylsulfonyl-L-phenylalanine(3-amidino)-4-(methylamidobuteryl)piperidide,
3-(4-amino-3-tetrahydropyridyl)phenylsulfonyl-L-phenylalanine(3-amidino)-4-(methylamidobuteryl)piperidide,
3-(Na-(CH3-PEG₁₀₀₀₀-CH₂-CH₂-CO)-α,ß-diaminopropionyl-NH)phenylsulfonyl-L-phenylalanine(3-amidino)-4-(2-Aminoethyl)-piperidide
3-(H-ß-alanyl-NH)phenylsulfonyl-L-phenylalanine(3-amidino)-4-(CH3-PEG₁₀₀₀₀-CH₂-CH₂-amidobuteryl)piperidide,
3-(CH₃-PEG₁₀₀₀₀-CH₂-CH₂-CO)-ß-Ala-NH)phenylsulfonyl-L-phenylalanine(3-amidino)-4-(2-aminoethyl)-piperidide,
3-(H-ß-alanyl-NH)phenylsulfonyl-L-phenylaianine(3-amidino)-nipecotyl-benzyl ester.

14. The compound of claim 7, wherein the compound is selected from one of the following structures:
3-(H-ß-alanine-NH)phenylsulfonyl-L-phenylalanine(3-aminomethyl)-4-(2-aminoethyl)piperidide,
3-(4-methoxyphenyl)phenylsulfonyl-L-phenylalanine(3-aminomethyl)-4-(2-aminoethyl)piperidide.

15. The compound of claim 9, wherein the compound is selected from one of the following structures:
3-(H-ß-alanyl-NH)phenylsulfonyl-L-phenylalanine(3-amidino)-4-(ethyloxycarbonyl)piperazide,
3-(H-ß-alanyl-NH)phenylsulfonyl-L-phenylalanine(3-amidino)-4-benzyloxycarbonyl)piperazide,
3-(H-ß-alanyl-NH)phenylsulfonyl-L-phenylalanine(3-amidino) piperazide,
3-(H-13-alanyl-NH)phenylsulfonyl-L-phenylalanine(3-aminomethyl)-4-(benzyloxycarbonyl)piperazide,
3-(H-ß-alanyl-NH)phenylsulfonyl-L-phenylalanine(3-AmidinoH-(CH3-PEG₁₀₀₀₀-CH₂-CH₂-NH-Suc)piperazide.

16. A medicinal product comprising at least one of the compounds according to any one of claims 1-15.

17. The medicinal product of claim 16 for the reduction of the formation of tumor metastases.

## Revendications

1. Composé de formule (I) 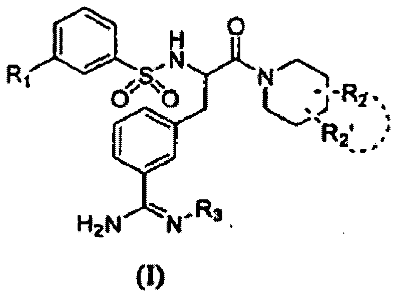 ou sel de ce composé,
dans lequel
R₁ est une structure cyclique éventuellement mono- ou multi-substituée choisie parmi un radical aryle ou hétéroaryle qui peuvent être partiellement ou complètement hydrogénés ; et
R₂ et R₂', s'ils sont présents seuls ou ensemble, sont indépendamment l'un de l'autre, un radical alkyle ramifié ou à chaîne linéaire comprenant 1 à 6 atomes de carbone dans lequel un ou plusieurs groupes méthylène peuvent être substitué(s) par des hétéroatomes ; un radical aminoalkyle ou guanidinoalkyle ramifié ou à chaîne linéaire comprenant 1 à 6 atomes de carbone ; ou -(CH₂)ₘ-C(=O)-NHR₄, où m est un nombre entier de 0 à 4 et R₄ désigne un atome d'hydrogène ou un radical -(CH₂)ₖ-CH₃ où k est un nombre entier de 0 à 3 ; ou R₂ et R₂' forment une structure cyclique qui forme conjointement avec le pipéridide, l'élément structural suivant : 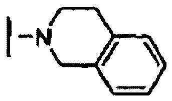 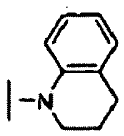 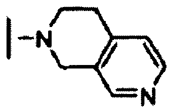 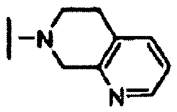 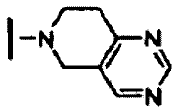 ou
R₁ est un radical aminoalkyle ramifié ou à chaîne linéaire, un radical hydroxyalkyle comprenant 1 à 6 atomes de carbone ; ou un radical 3-azétidine-C(=O)-NH ; et
R₂ et R₂', s'ils sont présents seuls ou ensemble, désignent indépendamment l'un de l'autre, un radical aminoalkyle ramifié ou à chaîne linéaire ou un radical guanidinoalkyle comprenant 1 à 6 atomes de carbone ; ou R₂ est -(CH₂)ₘ-C(=O)-NHR₄, où m est un nombre entier de 0 à 4 et R₄ désigne un atome d'hydrogène ou un radical -(CH₂)ₖ-CH₃ où k est un nombre entier de 0 à 3;
ou
R₁ est un radical H₂N-(CH₂)ₙ-C(=O)-NH, un radical HO-(CH₂)ₙ-C(=O)-NH où n est un nombre entier de 1 à 4 ; ou un radical 3-azétidine-C(=O)-NH ; et
R₂ et R₂', s'ils sont présents seuls ou ensemble, désignent indépendamment l'un de l'autre, un radical non basique : ou R₂ et R₂' forment une structure cyclique qui forme conjointement avec le pipéridide, l'un des éléments structuraux figurant ci-dessus ;
et
R₃ est, dans tous les cas ci-dessus, un atome d'hydrogène, un groupe hydroxyle, un groupe alcoxy, un groupe acétyloxy ou un groupe alkyloxycarbonyle, le radical alkyle comprenant 1 à 6 atomes de carbone.

2. Composé selon la revendication 1, dans lequel R₂' n'est pas présent, **caractérisé en ce que**
R₁ est choisi parmi un radical phényle, radical pyridyle, radical pyrimidine, radical indole, radical tétrahydropyridyle, radical pipéridinone ou radical pyridazinone éventuellement mono- ou multi-substitué ; et
R₂ est un radical aminoalkyle à chaîne linéaire comprenant 1 à 6 atomes de carbone; ou -(CH₂)ₘ-C(=O)-NHR₄, où m est un nombre entier de 0 à 4 et R4 désigne un atome d'hydrogène ou un radical -(CH₂)ₖ-CH₃ où k est un nombre entier de 0 à 3; ou
R₁ et R₂ sont un radical amino-alkyle à chaîne linéaire comprenant 1 à 6 atomes de carbone ;
ou
R₁ est un radical H₂N-(CH₂)ₙ-C(=O)-NH, où n est un nombre entier de 1 à 4;
et
R₂ est choisi parmi les radicaux suivants :
-(CH₂)ₘ-C(=O)-NHR₄, où m est un nombre entier de 0 à 4 et R₄ désigne un atome d'hydrogène ou un radical -(CH₂)ₖ-CH₃ où k est un nombre entier de 0 à 3 ;
ou -(CH₂)ₒ-C(=O)-OR₅ où o est un nombre entier de 1 à 6 et R₅ est un atome d'hydrogène ou un groupe alkyle à chaîne linéaire comprenant 1 à 4 atomes de carbone ;
et
R₃ est, dans tous les cas ci-dessus, un atome d'hydrogène, un groupe hydroxyle, un groupe alcoxy, un groupe acétyloxy ou un groupe alkyloxycarbonyle dans lequel le radical alkyle comprend 1 à 6 atomes de carbone.

3. Composé selon la revendication 1 ou 2, **caractérisé en ce que** le radical R₂ se trouve en position méta- ou para.

4. Composé selon l'une des revendications 1 à 3, **caractérisé en ce que** la substitution sur la structure cyclique du radical R₁ est choisie parmi les radicaux suivants : R₆-O où R₆ est un radical alkyle ramifié ou à chaîne linéaire comprenant 1 à 6 atomes de carbone ; (CH₃)-(CH₂)ₚ- où p est un nombre entier de 0 à 6 ; un atome d'halogène ; et/ou un groupe amino.

5. Composé selon l'une des revendications 1 à 4, **caractérisé en ce que** la substitution sur le radical phényle est choisie parmi les radicaux suivants : R₆-O où R₆ est un radical alkyle ramifié ou à chaîne linéaire comprenant 1 à 6 atomes de carbone ; (CH₃)-(CH₂)ₚ- où p est un nombre entier de 0 à 6 ; et/ou un atome d'halogène.

6. Composé selon l'une des revendications 1 à 4, **caractérisé en ce que** la substitution sur le radical pyridyle, le radical pyrimidine ou le radical tétrahydropyridyle est choisie parmi les radicaux suivants : (CH₃)-(CH₂)ₚ- où p est un nombre entier de 0 à 6 ;et/ou un groupe amino.

7. Composé de formule (II) 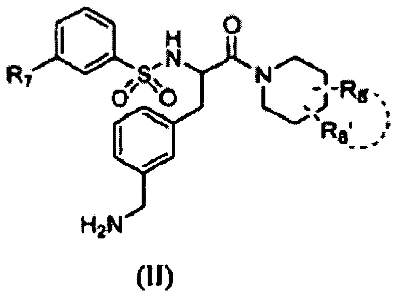 ou un sel de ce composé, dans laquelle
R₇ est une structure cyclique éventuellement mono- ou multi-substituée choisie parmi un radical alkyle ou un radical hétéroaryle qui est éventuellement partiellement ou complètement hydrogéné ; un radical alkyle ramifiée ou non ramifié comprenant 1 à 8 atomes de carbone dans lequel un ou plusieurs groupes méthylène peuvent être substitués par des hétéroatomes ; un radical H₂N-(CH₂)ᵣ-C(=O)-NH, un radical HO-(CH₂)ᵣ-C(=O)-NH, un radical NH₂-(CH₂)_{q} ou un radical HO-(CH₂)_{q}, où r est un nombre entier de 1 à 4 et les q indépendamment les uns des autres sont un nombre entier de 1 à 5 ; ou un radical 3-azétidine-C(=O)-NH ; et
R₈ et R₈'; s'ils sont présents seuls ou ensemble, sont indépendamment l'un de l'autre, un radical alkyle à chaîne linéaire comprenant 1 à 8 atomes de carbone, dans lequel un ou plusieurs groupes méthylène peuvent être substitués par des hétéroatomes ; un radical aminoalkyle ou un radical guanidinoalkyle comprenant respectivement 1 à 6 atomes de carbone ; ou -(CH₂)ₘ.C(=O)-NHR4, où m est un nombre entier de 0 à 4 et R₄ désigne un atome d'hydrogène ou un radical -(CH₂)ₖ-CH₃ où k est un nombre entier de 0 à 3 ; ou R₈ et R₈' forment une structure cyclique qui forme conjointement avec le pipéridide, un élément structural.

8. Composé selon la revendication 7, dans lequel R₈' n'est pas présent, **caractérisé en ce que**
R₇ est une structure cyclique éventuellement mono- ou multi-substituée choisie parmi un radical phényle, un radical pyridyle, un radical pyrimidine, un radical indole, un radical tétrahydropyridyle, un radical pipéridinone ou un radical pyridazinone ; un radical H₂N-(CH₂)ᵣ-C(=O)-NH, où r est un nombre entier de 1 à 4 ; et
R₈ est un radical aminoalkyle comprenant respectivement 1 à 6 atomes de carbone.

9. Composé de formule (III) 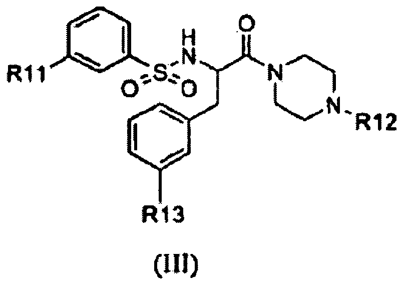 ou un sel de ce composé, dans laquelle
R₁₁ est un radical NH₂-(CH₂)ᵥ-C(=O)-NH où v est un nombre entier de 1 à 5 ; un radical 3-azétidine-C(=O)-NH, une structure cyclique éventuellement mono- ou multi-substituée choisie parmi un radical aryle ou un radical hétéroaryle qui peuvent être partiellement ou complètement hydrogénés ;
R₁₂ est un atome d'hydrogène ; un radical aminoalkyle ramifié ou à chaîne linéaire comprenant 1 à 6 atomes de carbone ; un groupe -C(=O)-O-R₁₄, un groupe -C(=O)-R₁₄ ou -C(=O)-NH-R₁₄, où les R₁₄ peuvent être respectivement, indépendamment les uns des autres, un radical alkyle comprenant 1 à 6 atomes de carbone ; ou un radical aralkyle ou un radical hétéroaralkyle comprenant respectivement 4 à 12 atomes de carbone dans leur segment aryle ou hétéroaryle, où le segment hétéroaryle du radical hétéro-aralkyle peut contenir 1 à 2 hétéroatomes, et comprenant respectivement 1 à 6 atomes de carbone dans le radical alkyle ; et
R₁₃ est un radical aminométhyle ou un radical amidino substitué ou non substitué de formule -C(=NR₁₅)NH₂ où R₁₅ est un atome d'hydrogène, un groupe hydroxyle, un groupe alcoxy, un groupe acétyloxy ou un groupe alkyloxycarbonyle, le radical alkyle comprenant 1 à 6 atomes de carbone.

10. Composé selon la revendication 9, **caractérisé en ce que** R₁₁ est un radical NH₂-(CH₂)ᵥ-C(=O)-NH où v est un nombre entier de 1 à 4 ;
R₁₂ est un atome d'hydrogène ; un radical aminoalkyle à chaîne linéaire comprenant 1 à 6 atomes de carbone ; ou -C(=O)-O-R₁₄, où les R₁₄ peuvent être indépendamment les uns des autres, un radical alkyle à chaîne linéaire comprenant 1 à 6 atomes de carbone ; ou un radical benzyle ; et
R₁₃ est un radical aminométhyle ou un radical amidino substitué ou non substitué de formule -C(=NR₁₅)NH₂ où R₁₅ est un atome d'hydrogène, un groupe hydroxyle, un groupe alcoxy, un groupe acétylhydroxy ou un groupe alkyloxycarbonyle où le radical alkyle comprend 1 à 6 atomes de carbone.

11. Composé selon au moins l'une des revendications 1 à 10, **caractérisé en ce que** une chaîne de polyéthylène glycol est couplée de manière covalente au composé, éventuellement au moyen d'un lieur.

12. Composé selon au moins l'une des revendications 1 à 11, **caractérisé en ce que** l'acide aminé en configuration L se présente comme un motif structural central dans les formules (I), (II) et (III).

13. Composé selon la revendication 1, où le composé est choisi parmi l'une des structures suivantes :
3-(3,4-diméthoxyphényl)phénylsulfonyl-L-phénylalanin(3-amidino)-4-(2-aminoéthyl)pipéridide,
3-(phényl)phénylsulfonyl-L-phénylalanin(3-amidino)-4-(2-aminoéthyl)pipéridide,
3-(4-méthoxyphényl)phénylsulfonyl-L-phénylalanin(3-amidino)-4-(2-aminoéthyl)pipéridide,
3-(4-éthoxyphényl)phénylsulfonyl-L-phénylalanin(3-amidino)-4-(2-aminoéthyl)pipéridide,
3-(4-éthylphényl)phénylsulfonyl-L-phénylalanin(3-amidino)-4-(2-aminoéthyl)pipéridide,
3-(4-isopropoxyphényl)phénylsulfonyl-L-phénylalanin(3-amidino)-4-(2-aminoéthyl)pipéridide,
3-(4-chlorophényl)phénylsulfonyl-L-phénylalanin(3-amidino)-4-(2-aminoéthyl)pipéridide,
3-(3-chlorophényl)phénylsulfonyl-L-phénylalanin(3-amidino)-4-(2-aminoéthyl)pipéridide,
3-(2-chlorophényl)phénylsulfonyl-L-phénylalanin(3-amidino)-4-(2-aminoéthyl)pipéridide,
3-(4-pyridyl)phénylsulfonyl-L-phénylalanin(3-amidino)-4-(2-aminoéthyl)pipéridide, 3-(3-pyridyl)phénylsulfonyl-L-phénylalanin(3-amidino)-4-(2-aminoéthyl)pipéridide,
3-(2-méthyl-4-pirimidinyl)phénylsulfonyl-L-phénylalanin(3-amidino)-4-(2-aminoéthyl)pipéridide,
3-(5-indolyl)phénylsulfonyl-L-phénylalanin(3-amidino)-4-(2-aminoéthyl)pipéridide,
3-(4-amino-3-pyridyl)phénylsulfonyl-L-phénylalanin(3-amidino)-4-(2-aminoéthyl)pipéridide,
3-(4-amino-3-tétrahydropyridyl)phénylsulfonyl-L-phénylalanin(3-amidino)-4-(2-aminoéthyl)pipéridide,
3-(1-aminobutyl)phénylsulfonyl-L-phénylalanin(3-amidino)-4-(2-aminoéthyl)pipéridide,
3-(H-β-alanyl-NH))phénylsulfonyl-L-phénylalanin(3-amidino)-4-(méthylamidobutéryl)pipéridide,
3-(4-éthoxyphényl)phénylsulfonyl-L-phénylalanin(3-amidino)-4-(méthylamidobutéryl)pipéridide,
3-(4-éthylphényl)phénylsulfonyl-L-phénylalanin(3-amidino)-4-(méthylamidobutéryl)pipéridide,
3-(H-β-alanyl-NH)phénylsulfonyl-L-phénylalanin(3-amidino)-4-pipéridinbutéryl-O-méthylate,
3-(H-β-alanyl-NH)phénylsulfonyl-L-phénylalanin(3-amidino)-4-(amidobutéryl) pipéridide,
3-(H-β-alanyl-NH)phénylsulfonyl-L-phénylalanin(3-amidino)-4-(butéryl)pipéridide,
3-(H-β-alanyl-NH)phénylsulfonyl-L-phénylalanin(3-amidino)-isonipecotylamide,
3-(H-β-alanyl-NH)phénylsulfonyl-L-phénylalanin(3-amidino)-nipécotylamide,
3-(H-β-alanyl-NH)phénylsulfonyl-L-phénylalanin(3-amidino)-pipéridide,
3-(pyridazinone)phénylsulfonyl-L-phénylalanin(3-amidino)-4-(méthylamidobutéryl) pipéridide,
3-(pipéridinone)phénylsulfonyl-L-phénylalanin(3-amidino)-4-(méthylamidobutéryl) pipéridide,
3-(pipérazinone)phénylsulfonyl-L-phénylalanin(3-amidino)-4-(méthylamidobutéryl) pipéridide,
3-(4-éthoxyphényl)phénylsulfonyl-L-phénylalanin(3-hydroxyamidino)-pipéridinebutéryl-N-méthylamide,
3-(4-éthylphényl)phénylsulfonyl-L-phénylalanin(3-hydroxyamidino)-pipéridinebutéryl-N-méthylamide,
3-(1-hydroxybutyl)phénylsulfonyl-L-phénylalanin(3-amidino)-4-(2-aminoéthyl)-pipéridide,
3-(3-azétidin-CONH)phénylsulfonyl-L-phénylalanin(3-amidino)-4-(méthylamidobutéryl)-pipéridide,
3-(3-azétidin-CONH)phénylsulfonyl-L-phénylalanin(3-hydroxyamidino)-4-(méthylamidobutéryl)-pipéridide,
3-(3-azétidin-CONH)phénylsulfonyl-L-phénylalanin(3-amidino)-4-(2-aminoéthyl)pipéridide,
3-(4-tert-butylphényl)phénylsulfonyl-L-phénylalanin(3-amidino)-4-(méthylamidobutéryl)-pipéridide,
3-(4-tert-butylphényl)phénylsulfonyl-L-phénylalanin(3-hydroxyamidino)-4-(méthylamidobutéryl)-pipéridide,
3-(4-tert-butylphényl)phénylsulfonyl-L-phénylalanin(3-méthoxyamidino)-4-(méthylamidobutéryl)-pipéridide,
3-(4-isopropylphényl)phénylsulfonyl-L-phénylalanin(3-amidino)-4-(méthylamidobutéryl)-pipéridide,
3-(4-méthoxy-3-pyridyl)phénylsulfonyl-L-phénylalanin(3-amidino)-4-(méthylamidobutéryl)-pipéridide,
3-(4-amino-3-pyridyl)phénylsulfonyl-L-phénylalanin(3-amidino)-4-(méthylamidobutéryl)-pipéridide,
3-(4-amino-3-tétrahydropyridyl)phénylsulfonyl-L-phénylalanin(3-amidino)-4-(méthylamidobutéryl)-pipéridide,
3-(Na-(CH₃-PEG₁₀₀₀₀-CH₂-CH₂-CO)-α,β-diaminopropionyl-NH)phénylsulfonyl-L-phénylalanin(3-amidino)-4-(2-aminoéthyl)-pipéridide,
3-(H-p-alanyl-NH)phénylsulfonyl-L-phénylalanin(3-amidino)-4-(CH₃-PEG₁₀₀₀₀-CH₂-CH₂-amidobutéryl)-pipéridide,
3-(CH₃-PEG₁₀₀₀₀-CH₂-CH₂-CO)-β-Ala-NH)phénylsulfonyl-L-phénylalanin(3-amidino)-4-(2-aminoéthyl)-pipéridide,
3-(H-β-alanyl-NH)phénylsulfonyl-L-phénylalanin(3-amidino)-nipécotyl-benzylester.

14. Composé selon la revendication 7, où le composé est choisi parmi l'une des structures suivantes :
3-(H-β-alanyl-NH))phénylsulfonyl-L-phénylalanin(3-aminométhyl)-4-(2-aminoéthyl)pipéridide,
3-(4-méthoxyphényl)phénylsulfonyl-L-phénylalanin(3-aminométhyl)-4-(2-aminoéthyl)pipéridide.

15. Composé selon la revendication 9, le composé étant choisi parmi l'une des structures suivantes :
3-(H-β-alanyl-NH))phénylsulfonyl-L-phénylalanin(3-amidino)-4-(éthyloxycarbonyl) pipérazide,
3-(H-β-alanyl-NH)phénylsulfonyl-L-phénylalanin(3-amidino)-4-(benzyloxycarbonyl) pipérazide,
3-(H-β-alanyl-NH)phénylsulfonyl-L-phénylalanin(3-amidino)-pipérazide,
3-(H-β-alanyl-NH)phénylsulfonyl-L-phénylalanin(3-aminométhyl)-4-(benzyloxycarbonyl)pipérazide,
3-(H-β-alanyl-NH)phénylsulfonyl-L-phénylalanin(3-amidino)-4-(CH3-PEG₁₀₀₀₀-CH₂-CH₂-NH-suc)pipérazide.

16. Médicament comprenant au moins l'un des composés selon l'une des revendications 1 à 15.

17. Médicament selon la revendication 16 pour la réduction de la formation de métastases tumorales.
